(19)

Europäisches
Patentamt
European
Patent Office
Office européen
des brevets

(11) **EP 3 808 355 A1**

(12) **EUROPEAN PATENT APPLICATION**
published in accordance with Art. 153(4) EPC

(43) Date of publication:
**21.04.2021   Bulletin 2021/16**

(51) Int Cl.:
***A61K 35/28*** (2015.01)    ***A61P 31/04*** (2006.01)

(21) Application number: **18922187.2**

(22) Date of filing: **15.06.2018**

(86) International application number:
**PCT/CL2018/050046**

(87) International publication number:
**WO 2019/237212 (19.12.2019 Gazette 2019/51)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Universidad De Chile
Santiago (CL)**

(72) Inventors:
• **PERALTA TRONCOSO, Oscar Alejandro
  La Reina, Santiago (CL)**
• **TORRES MENDOZA, Cristian Gabriel
  La Florida,  Santiago (CL)**

(74) Representative: **Zehetner Patentanwälte
Karmeliterstraße 6
52064 Aachen (DE)**

(54) **FOETAL MESENCHYMAL STEM CELL COMPOSITION OF ALLOGENIC ORIGIN, TREATMENT METHOD AND USE THEREOF IN MASTITIS IN MILK-PRODUCING ANIMALS, INCLUDING CATTLE**

(57)    The present invention relates to the use of regenerative medicine in livestock farming, in particular to regenerative medicine for the care of livestock, and even more particularly to regenerative medicine in the care of milk-producing animals, including cattle. Specifically, the invention concerns a foetal mesenchymal stem cell (MSC) composition of allogenic origin, a treatment method and the use thereof in mastitis in milk-producing animals, including cattle, suffering from mastitis, to immunostimulate the animal, the MSC coming from foetal bovine adipose tissue. The composition can be administered via the intramammary route, exercising an immunomodulatory effect on the inflammatory response and strengthening antimicrobial activity against mastitis-causing pathogens in milk-producing animals with mastitis, thereby becoming an alternative to replacing and/or supplementing the use of antibiotics.

EP 3 808 355 A1

**Description**

**FIELD OF THE INVENTION**

The present invention relates to the application of regenerative medicine in livestock, especially with regenerative medicine for the care of cattle, and even more especially, with regenerative medicine in the care of milk-producing animals, including bovines. In particular, the invention relates to a composition of fetal mesenchymal stem cells (MSC) of allogeneic origin, treatment method and its use in mastitis in milk-producing animals, including bovines, suffering from mastitis, and to immunostimulate said animal, where MSCs come from fetal bovine adipose tissue. The composition can be administered intramammary, exerting an immunomodulatory effect on the inflammatory response and strengthening the antimicrobial activity against pathogens that cause mastitis in milk-producing animals with mastitis and becoming an alternative for the replacement and / or complementation of the use of antibiotics.

**BACKGROUND**

The dairy industry is a business with high economic impact that delivers nutritional products to different age groups globally and enables the economic support of medium to small-scale producers in developing countries.
To a large extent, this industry relies on the production potential of the bovine mammary gland, which is capable of producing <8000kg of milk per lactation. Inflammation of the mammary gland or mastitis, generally as a consequence of infectious agents, causes damage to the mammary tissue, reducing the number and activity of epithelial cells, consequently decreasing milk production. Mastitis is the disease with the highest prevalence and economic cost among dairy cattle worldwide. In studies carried out in farms in the central zone of Chile, prevalence of mastitis (clinical and subclinical) has been determined and varies between 65 and 88%. Meanwhile, an economic cost associated with mastitis for the dairy industry has been estimated at approximately USD $ 2 billion annually. In addition to the economic losses, mastitis has been declared as one of the main pathologies that threaten animal welfare. Bovine mastitis also affects public health by producing physical and usually microbiological changes in milk, as well as increasing the risk for the presence of residues. Nowadays, despite the improvements that have been implemented in milking hygiene, as well as in the general management of dairy cattle, mastitis is still the most prevalent pathology that generates greater economic losses for both the producer and the dairy industry. The treatment and prevention of mastitis are complex because of bacterial multi-causality. In order to control the growth of the bacteria responsible for the infection of the mammary gland the main treatment and prevention strategy involves the use of antibiotics such as cephalosporins, penicillins, streptomycins and tetracyclines. However, this treatment is not entirely effective and its improper use has been associated to the increase in the resistance of pathogenic bacteria and a greater risk of antibiotics presence in milk. Consequently, recent evidence has emerged indicating that the use of antibiotics in agriculture would be responsible for the increase in bacterial resistance and would affect the efficacy of these antibiotics in humans.
The presence of these antibiotics in milk not only represents a penalty for the dairy producer but is also of great importance for public health and the safety of milk for human consumption. On the other hand, antibiotic treatment does not address the regeneration of mammary glandular tissue, the integrity of which is critical in terms of milk production. Additionally, in recent years a series of vaccines have been developed to control bovine mastitis. These types of vaccines have mitigated the severity of the infection, however, they have not shown significant effects in preventing the presentation of new cases given a high variation in the response to immunization between animals.
Comparing antibiotics and vaccines, it is possible to indicate that the former have advantages including a high cost-benefit ratio and a high effectiveness in pathogens other than Staphylococcus aureus (S. aureus). Some disadvantages of antibiotics are a low effectiveness in mastitis caused by S. aureus, the potential risk of generating resistance from pathogens, generating residues in the milk, the requirement of a period of storage or lack of milk and the lack of a regenerative effect of the mammary gland. Meanwhile, vaccines have advantages including their low cost, the ability to reduce the severity of the condition and the associated costs of treatment; whereas, disadvantages include their low effectiveness in preventing the presentation of new cases and the presence of a maximum limit of protection .
Stem cells have been a focus of intense research during the last decades, changing the perspective of development, pathophysiology and treatment of various diseases (Joseph NM, Morrison SJ. 2005. Toward an understanding of the physiological function of mammalian stem cells. Dev Cell 9, 173-183; Clarke MF, Fuller M. 2006. Stem cells and cancer: two faces of eve. Cell 124, 1111-1115). Stem cells are commonly defined as cells with unlimited capacity for self-renewal and differentiation towards all or some cell types of a tissue or organ, and are categorized into embryonic stem cells (ESC) and adult stem cells, among which, best known correspond to mesenchymal stem cells (MSCs). MSCs possess limited differentiation potential to mesenchymal (multipotent) cell lineages compared to ESCs that possess broader (pluripotent) differentiation potential (Jaenisch R, Young R. 2008. Stem cells, the molecular circuitry of pluripotency and nuclear reprogramming. Cell 132, 4, 567-582). The main function of adult stem cells is to form specialized cells in a living organism and to maintain and repair the tissue in which they are found. They are defined by their ability to self-renew, generate differentiated progeny, and express specific molecular markers (Caplan Al. 1991. Mesenchymal stem cells. J Orthop Res 9, 5, 641-650). The main in vivo function of bone marrow-derived MSCs is to enhance the formation of hematopoietic cells and contribute to bone repair (Pittinger et al., 1999). These cells represent a very low proportion of the bone marrow cell population, with values ranging from 0.001 to 0.01% (Pittenger MF, Mackay AM, Beck SC, Jaiswal RK, Douglas R, Mosca JD, Moorman MA, Simonetti DW, Craig S, Marshak Dr. 1999. Multilineage potential of adult human mesenchymal stem cells. Science 284, 541 1, 143-147). MSCs have been derived from different species mainly based on their ability to adhere to culture plates and the identification of their fibroblast morphology (Cortes Y, Ojeda M, Araya D, Dueñas F, Fernandez MS, Peralta OA. 2013. Isolation and multilineage differentiation of bone marrow mesenchymal stem cells from abattoir-derived bovine fetuses. BMC Vet Res 9, 133, 1-14). Once isolated, MSC expansion requires a significant culture time ranging from 3 to 6 weeks. (Godwin EE, Young NJ, Dudhia J, Beamish IC, Smith RK. 2012. Implantation of bone marrow-derived mesenchymal stem cells demonstrates improved outcome in horses with overstrain injury of the superficial digital flexor tendon. Equine Vet J 44, 25-32). Subsequently, it is possible to evaluate their differentiation capacity through in vitro induction towards mesodermal lineages such as osteocytes, chondrocytes and adipocytes (Cortes Y, Ojeda M, Araya D, Dueñas F, Fernandez MS, Peralta OA. 2013. Isolation and multilineage differentiation of bone marrow mesenchymal stem cells from abattoir-derived bovine fetuses. BMC Vet Res 9, 133, 1-14). The ability to adhere to plastic, the potential for mesodermal differentiation, the expression of mesenchymal markers (CD73, CD90 and CD105) and the absence of hematopoietic markers (CD45, CD34 and CD14), are the criteria established by the International Society for Cell Therapy for the definition of a human MSC population (Dominici M, Le Blanc K, Mueller I, Slaper-Cortenbach I, Marini FC, Krause DS, Deans RJ, Keating A, Prockop DJ, Horwitz EM: Minimal criteria for defining multipotent mesenchymal stromal cells. The international society for cellular therapy position statement. Cytotherapy 2006, 8, 315-317). On the other hand, a MSC population has been described in adipose tissue that can be isolated by enzymatic digestion and subsequent filtration. The adherence to the plastic and its subsequent expansion allows obtaining relatively homogeneous MSC cultures. MSCs from adipose tissue express the major mouse and human class I histocompatibility complex but not class II. Yanez R, Lamana ML, García-Castro J, Colmenero I, Ramírez M, Bueren J A. Stem Cells 2006, 24, 2582-2591. Adipose tissue-derived mesenchymal stem cells have in vivo immunosuppressive properties applicable for the control of the graft-versus-host disease. Stem Cells 24, 2582-2591). Additionally, MSCs derived from human adipose tissue do not stimulate the proliferation of lymphocytes, but rather inhibit the proliferation of T cells and stimulate peripheral mononuclear cells in the blood, which indicates that these cells would not cause an immune response if used in autologous therapy (Minteer DM, Marra KG, Rubin JP. 2015. Adipose stem cell: biology, safety, regulation and regenerative potential. Clin Plast Surg 42, 2, 169-179). Adipose tissue MSCs have been differentiated into osteogenic, adipogenic and chondrogenic lineages (Gronthos S, Franklin DM, Leddy FIA, Robey PG, Storms RW, Gimble JM. 2001. Surface protein characterization of human adipose tissue-derived stromal cells. J Cell Physiol 189, 54-63; Katz AJ, Tholpady A, Tholpady SS, Shang H, Ogle RC. 2005. Cell surface and transcriptional characterization of human adipose-derived adherent stromal (hADAS) cells. Stem Cells 23, 412-423; Zhu Y, Liu T, Song K, Ning R, Ma X, Cui Z. 2009. ADSCs differentiated into cardiomyocytes in cardiac microenvironment. Mol Cell Biochem 324, 1 17-129). In comparative studies carried out between MSC of bone marrow and adipose tissue, it has been reported that the latter are less efficient in terms of their capacity for osteogenic and chondrogenic differentiation, despite their great similarity in relation to the pattern of gene expression during the differentiation process. (Liu TM, Martina M, Hutmacher DW, Huí JH, Lee EH, Lim B. 2007. Identification of common pathways mediating differentiation of bone marrow- and adipose tissue-derived human mesenchymal stem cells into three mesenchymal lineages. Stem Cells 25, 750-760; Yoshimura H, Muneta T, Nimura A, Yokoyama A, Koga H, Sekiya I. 2007. Comparison of rat mesenchymal stem cells derived from bone marrow, synovium, periosteum, adipose tissue, and muscle. Cell Tissue Res 327, 449-462). In bovine species, there are still no studies that allow determining differences between MSCs derived from bone marrow or adipose tissue.

Regarding the population of stem cells in the mammary gland, there is a large amount of information available in relation to progenitor cells of the mouse and human mammary gland, in studies that range from biological characterization to their possible role in cancer (Bruno RD, Smith GH. 201 1. Role of epithelial stem / progenitor cells in mammary cancer. Gene Expr 15, 133-140; VanKeymeulen et al., 201 1; Pond AC, Bin X, Batts T, Roarty K, Hilsenbeck S, Rosen JM. 2013. Fibroblast growth factor receptor signalling is essential for normal mammary gland development and stem cell function. Stem Cells 32, 178-189). In contrast, there is limited information regarding stem cells and their progeny in the bovine mammary gland. In this regard, the existence of a population of bovine mammary stem cells (MaSC) has been reported that would fulfil the function of generating common cellular progenitors with the capacity to differentiate into luminal and myoepithelial epithelial progenitor cells (Capuco AV, Choudhary RK, Daniels KM, Li RW, Evock-Clover CM. 2012. Bovine mammary stem cells: cell biology meets production agriculture. Animal 6, 382-393). This cellular hierarchy plays a fundamental role, especially in the postnatal development of the mammary gland, since it allows its growth from birth (Capuco AV, Akers RM, Smith JJ. 1997. Mammary growth in Holstein cows during the dry period: quantification of nucleic acids and histology. J Dairy Sci 80, 477-487), the epithelial cells being important participants in the defense mechanisms against breast infection (Wellnitz O, Kerr DE. 2004. Cryopreserved bovine mammary cells to model epithelial response to infection. Vet Immunol Immunopathol 101, 191-202). Due to its regenerative potential, the use of MSC could have a strong and positive impact on the repair of damaged udder tissue, including epithelial, myoepithelial and stromal tissue. So far, there are two cell types, epithelial and myoepithelial progenitors, which are of considerable interest for their regeneration by therapeutic manipulation. Therefore, the expansion in the number of MSC through its pharmacological induction or its replacement by cell therapy with MSC has interesting potential applications both for the regeneration of damaged tissue, the increase in the milk production capacity and also its application in the production of proteins for pharmacological application in milk.

Regarding allogeneic therapies using fetal MSCs, it is important to note that most of the clinical studies reported in Veterinary Medicine are based on the use of MSC as autologous therapy (Webster RA, Blaber SP, Herbert BR, Wilkins MR, Vesey G 2012. The role of mesenchymal stem cells in veterinary therapeutics - a review. N Z Vet J 60, 5, 265-272). In this type of treatment, cells are collected and applied for treatment in the same donor. Autologous MSC collection is a relatively simple process, but requires specialized equipment and infrastructure, and isolation and expansion of cultures can take from days to weeks (Schnabel LV, Fortier LA, McIlwraith CW, Nobert KM. 2013. Therapeutic use of stem cells in horses: which type, how, and when? Vet J 197, 3, 570-577). This period of cell expansion can generate a delay in the application of the treatment (4 to 6 weeks) that in some cases is highly limiting for the success of the therapy. MSCs express low levels of major histocompatibility complex (MHC) I and II antigens and lack T-lymphocyte costimulatory molecules (CD80 and CD86) (Patel et al., 2008). These properties allow MSCs to be recognized as "immune-evasive" cells, being able to avoid their detection by the immune system and therefore serve for allogeneic therapy (Aggarwal S, Pittenger MF. 2005. Human mesenchymal stem cells modulate allogenic immune cell responses. Blood 105, 1815-1822). Therefore, obtaining MSC from donors can allow the generation of a cell stock of immediate bioavailability for use. The use of allogeneic sources makes it possible to have a bank or permanent storage of MSCs for its therapeutic use and therefore avoid the time spent on extraction, isolation and expansion of MSCs, which is required in autologous therapies. For this purpose, the development of cell cryopreservation banks is required to facilitate the transport and immediate availability of cells. On the other hand, the use of MSCs obtained from fetuses presents a series of advantages in relation to other sources of MSCs. The availability of MSCs decreases during the growth of individuals, so the possibility of having isolated MSC from adult individuals decreases and is diametrically less than the fetal stage. In humans, this decrease has been calculated to range from 0.001 to 0.0004% before age 30 to 0.00025 to 0.00005% after age 60 (Caplan AI. 2007. Adult mesenchymal stem cells for tissue engineering versus regenerative medicine. J Cell Physiol 213, 341-317). Additionally, MSCs isolated from adult individuals have lower differentiation potential compared to fetal MSCs (Zhang ZY, Teoh SH, Chong MS, Schantz JT, Fisk NM, Choolani MA, Chan J. 2009. Superior osteogenic capacity for bone tissue engineering of fetal compared with perinatal and adult mesenchymal stem cells. Stem Cells, 27, 126-137). Furthermore, MSCs extracted from adult individuals have been more exposed to diseases in the donor that can affect their proliferative and differentiation potential. Fetal MSCs have also been described as having a lower immunogenic capacity compared to adult MSCs due to lower expression of noncytosolic MHC I and II (Le Blanc K, Tammik L, Sundberg B, Haynesworth SE, Ringdén O. 2003. Mesenchymal stem cells inhibit and stimulate mixed lymphocyte cultures and mitogenic responses in dependently of the major histocompatibility complex. Scand J Immunol 57, 1 1-20; Zhang ZY, Teoh SH, Chong MS, Schantz JT, Fisk NM, Choolani MA, Chan J. 2009. Superior osteogenic capacity for bone tissue engineering of fetal compared with perinatal and adult mesenchymal stem cells. Stem Cells, 27, 126-137).

The properties of MSCs for the treatment of clinical disorders are known, and a large number of studies have reported the therapeutic application of MSC for the treatment of various clinical disorders, including myocardial infarction (Lee RH, Pulin AA, Seo MJ, Kota DJ, Ylostalo J, Larson BL, Semprun-Prieto L, Delafontaine P, Prockop DJ. 2009. Intravenous HMSCs improve myocardial infarction in mice because cells embolized in lung are activated to secrete the anti-inflammatory protein TSG-6. Cell Stem Cell 5, 54-63), diabetes (Lee RH, Seo MJ, Reger RL, Spees JL, Pulin AA, Olson SD, Prockop DJ. 2006. Multipotent stromal cells from human marrow home to and promote repair of pancreatic islets and renal glomeruli in diabetic NOD / scid mice. Proc Nati Acad Sci USA 103, 17438-17443), sepsis (Nemeth K, Leelahavanichkul A, Yuen PS, Mayer B, Parmelee A, Doi K, Robey PG, Leelahavanichkul K, Koller BH, Brown JM, Hu X, Jelinek I, Star RA, Mezey E. 2009. Bone marrow stromal cells attenuate sepsis via prostaglandin e (2) -dependent reprogramming of host macrophages to increase their interleukin-10 production. Nat Med 15: 42-49), liver deficiency (Parekkadan B, van Poli D, Suganuma K, Carter EA, Berthiaume F, Tilles AW, Yarmush ML. 2007. Mesenchymal stem cell-derived molecules reverse fulminant hepatic failure. PLoS One 2, e941), acute renal failure (Togel F, Hu Z, Weiss K, Isaac J, Lange C, Westenfelder C. 2005. Administered mesenchymal stem cells protect against ischemic acute renal failure through differentiation-independent mechanisms. Am J Physiol Renal Physiol, 289, F31-F42) and lung disease (Xu J, Qu J, Cao L, Sai Y, Chen C, Fie L, Yu L. 2008. Mesenchymal stem cell-based angiopoietin-1 gene therapy for acute lung injury induced by lipopolysaccharide in mice. J Pathol 214, 472-481). Several properties make the use of MSCs attractive for infectious pathologies. The ability to migrate to the sites of tissue damage, a property known as "homing", is one of these properties (Rojas M, Xu J, Woods CR, Mora AL, Spears W, Roman J, Brigham KL. 2005. Bone marrow-derived mesenchymal stem cells in repair of the injured lung. Am J Respir Cell Mol Biol. 33, 145-152). After their systemic or local administration, MSCs are attracted by chemotactic factors generated at the site of tissue damage (Kidd S, Spaeth E, Dembinski JL, Dietrich M, Watson K, Klopp A, Battula VL, Weil M, Andreeff M, Marini FC. 2009. Direct evidence of mesenchymal stem cell tropism for tumor and wounding microenvironments using in vivo bioluminescent imaging. Stem Cells 27, 2614-2623). MSCs express a series of receptors for some growth factors such as PDGF and IGF-1 and chemokines such as CCR2, CCR3, CCR4 and CCL5 (Ponte AL, Marais E, Gallay N, Langonne A, Delorme B, Herault O, Charbord P, Domenech J. 2007. The in vitro migration capacity of human bone marrow mesenchymal stem cells: comparison of chemokine and growth factor chemotactic activities. Stem Cells 25, 1737-1745). The interaction between MSCs and endothelial cells of blood vessels would be mediated by several surface integrins such as Integrin a4 / p1, which mediates cell-cell and cell-extracellular matrix interactions by adhering to the molecule (VCAN) -1 and the region V for fibronectin, respectively (Lewinsohn DM, Bargatze RF, Butcher EC. 1987. Leukocyte-endothelial cell recognition: evidence of a common molecular mechanism shared by neutrophils, lymphocytes, and other leukocytes. J Immunol 138, 4313-4321).

Additionally, MSCs have the capacity to modulate the immune response as has been reported in treatments carried out in patients with Crohn's disease (García-Olmo et al., 2005). Immunosuppressive function could be associated with the blocking of inflammatory molecules such as IL6, IL10, MMPs and nitric oxide (Jarvinen L, Badri L, Wettlaufer S, Ohtsuka T, Standiford TJ, Toews GB, Pinsky DJ, Peters-Golden, M, Lama VN 2008. Lung resident mesenchymal stem cells isolated from human lung allografts inhibit T cell proliferation via a soluble mediator. J Immunol 181, 4389-4396). Alternatively, MSCs can stimulate the immune response by producing pro-inflammatory cytokines (Rasmusson I, LeBlanc K, Sundberg B, Ringdén O. 2007. Mesenchymal stem cells stimulate antibody secretion in human B cells. Scand J Immunol 65, 336-343). This dual immunoregulatory function could be dependent on cell concentration (Le Blanc K, Tammik L, Sundberg B, Haynesworth SE, Ringdén O. 2003. Mesenchymal stem cells inhibit and stimulate mixed lymphocyte cultures and mitogenic responses in dependently of the major histocompatibility complex. Scand J Immunol 57, 11-20; Guest DJ, Smith MR, Alien WR. 2008. Monitoring the fate of autologous and allogenic mesenchymal progenitor cells injected into the superficial digital flexor tendon of horses: preliminary study. Equine Vet 40, 178-181). Regarding regenerative capacity, MSCs have shown potential for multilineage differentiation in in vitro studies (Cortes Y, Ojeda M, Araya D, Dueñas F, Fernandez MS, Peralta OA. 2013. Isolation and multilineage differentiation of bone marrow mesenchymal stem cells from abattoir-derived bovine fetuses. BMC Vet Res 9, 133, 1-14). The potential for transdifferentiation and implantation has been reported in vivo (Krause DS, Theise ND, Collector MI, Henegariu O, Hwang S, Gardner R, Neutzel S, Sharkis SJ. 2001. Multi-organ, multi- lineage engraftment by a single bone marrow-derived stem cell. Cell 105, 369-377); however, its regenerative effect is currently considered to be based mainly on the secretion of paracrine factors that stimulate the activity of host cells (Fang X, Neyrinck AP, Matthay MA, Lee JW. 2010. Allogeneic human mesenchymal stem cells restore epithelial protein permeability in cultured human alveolar type ii cells by secretion of angiopoietin-1. J Biol Chem 285, 2621 1-26222). Additionally, MSCs have been reported to have an antimicrobial potential mediated by paracrine factors such as keratinocyte growth factor (KGF), antimicrobial peptides, Angiopoietin 1 (ANG1), Interleukin 1 (IL1) and Prostaglandin E2 (PGE2), in addition to stimulation of macrophage and monocyte phagocytosis (Lee JW, Fang X, Krasnodembskaya A, Howard JP, Matthay MA. 2011. Concise review: Mesenchymal stem cells for acute lung injury: role of paracrine soluble factors. Stem Cells 29, 6, 913-919). In general, terms, the properties of migration, immunomodulation and regeneration would be of substantial importance for its therapeutic application in lesions produced by infectious agents. In the case of mastitis, the intramammary administration of MSC would allow these cells to be distributed in the places of injury of the mammary gland where they would exert an immunomodulatory effect on the immune response, antimicrobial against the pathogen and subsequently regenerative of the damaged glandular tissue. This cell therapy could replace or complement the antimicrobial effect of antibiotics in mastitis, making it possible to avoid or reduce their use or alternatively to reinforce their effect.

It is known for bovine mastitis that S. aureus is one of its main causing pathogens and that it produces a number of virulence factors including hemolysin, coagulase, and leukocidin (Sutra L, Poutrel B. 1994. Virulence factors involved in the pathogenesis of bovine intramammary infections due to Staphylococcus aureus. J Med Microbiol 40, 79-89). Persistent infection with S. aureus has been associated with a deficient immune response as a consequence of virulence factors. Its intramammary infection generates a chronic condition with great potential for resistance to antibiotic therapy. In national studies, high resistance of S. aureus (24.7-38.9% of isolates resistant) to various antibiotics including Penicillin, Amoxicillin and Streptomycin has been reported (San Martin et al., 2002). In the southern zone of Chile, which comprises most of the national herds, contagious mastitis occurs more frequently, the main agent of which is S. aureus (San Martin et al., 2002). Inoculation of 900 colony-forming units (CFUs) of S. aureus through the nipple canal has been reported to induce clinical signs in each infected quarter and a peak in somatic cell count (SCC) of 1400 x 103 (Talbot BG, Lacasse P. 2005. Progress in the development of mastitis vaccines. Livestock Prod Sci 98, 101 -113).

In particular, in the prior state of the art it is possible to mention the publication of the International Journal of Biological Sciences 2013; 9 (8): 818-829. doi: 10.7150 / ijbs.6901 Research Paper Stem Cell Research: A Novel Boulevard towards Improved Bovine Mastitis Management Neelesh Sharma et al. Received 2013.06.13; Accepted: 2013.08.02; Published: 2013.08.20, which refers to clinical mastitis and its effects on tissues, animal welfare and dairy productivity. It proposes the use of mammary epithelial cells MECs (inner layer or luminal epithelial cells) and their stem cells; and an outer layer of myoepithelial cells. The inner layer of bovine MECs produces CK18 and CK19 and the outer layer CK14, a-SMA and p63. It indicates that the results obtained in humans and mice are encourage by the application of stem cells in bovines. Thus, he proposes the use of stem cells to correct the structural and cytological defects caused by mastitis in the udder of bovines. It reviews several types of stem cells that would have therapeutic utility and their potential use in therapies to manage post-mastitis damage in bovines and thus restore milk production. It is argue that the possibility of bovine mammary stem cell therapy would allow significant tissue regeneration and replace or repair diseased or damaged tissue by differentiation into epithelial, myoepithelial and / or cuboidal / columnar cells in the udder, with minimal risk of rejection and collateral effects.

Fetal Stem Cells in Farm Animals: Applications in Health and Production PS Yadav · RK Singh · B. Singh Received: 15 October 2011 / Accepted: 5 December 201 1 / Published online: 19 January 2012 NAAS (National Academy of Agricultural Sciences) 2012 (Agrie Res (January-March 2012) 1 (1): 67-77 DOI 10.1007 / s40003- 01 1 -0001 -7) refers to scientifically validated embryonic stem cell lines (ES) in species other than mice, humans or some primates. It performs a review of the biological properties and easy derivation of fetal or adult stem cells that play a vital role in the health and production of livestock. They note that the placenta and fetal adnexa such as umbilical cord blood, umbilical cord matrix, fetal fibroblasts, and amniotic cells are an immensely valuable source of progenitor and pluripotent cell lineages. A large number of fetal cells are accessible, which can have various applications including assisted reproduction, regenerative medicine, or induced pluripotent stem cell production. It indicates that since fetal stem cells have markers of pluripotency expression and growth kinetics similar to ES cells, it is possible to think that they may be homologous to ES cells.

US 20140134140 A1 describes a method based on the use of human MSCs for the treatment of cystic fibrosis challenged with bacteria such as Pseudomona aeruginosa, Staphylococcus and Streptococcus pneumoniae.

The United States Food and Drug Administration (FDA) has approved the product "Hemacord" as a stem cell-based therapy in patients suffering from disorders of the hematopoietic system. The FDA has also approved for clinical phase I research, an MSC neural progenitor-based therapy for the treatment of multiple sclerosis in humans. The FDA also approved the production of placental-derived stem cells for the production of the product "Pluristem Placental Expanded", with potential applications for the treatment of aplastic anemia and muscle injuries. The Canadian Food and Drug Administration has approved the PROCHYMAL product for stem cell-based therapy in immune rejection diseases in children suffering from post-bone marrow transplant complications in graft versus host disease (GVHD)..

The present invention carried out a series of in vitro studies with MSCs isolated from fetal bovine bone marrow to induce osteogenic, chondrogenic and adipogenic differentiation of these cells (Cortes Y, Ojeda M, Araya D, Dueñas F, Fernandez MS, Peralta OA. 2013.Isolation and multilineage differentiation of bone marrow mesenchymal stem cells from abattoir-derived bovine fetuses. BMC Vet Res 9, 133, 1 - 14), demonstrating the ability of MSCs to differentiate into hepatogenic and neurogenic lineages (Dueñas F, Becerra V, Cortes Y, Vidal S, Sáenz L, Palomino J, De los Reyes M, Peralta OA. 2014. Hepatogenic and neurogenic differentiation of bone marrow mesenchymal stem cells from abattoir-derived bovine fetuses. BMC Vet Res 10, 154, 1 -13), and the expression potential of the GFP reporter gene in bovine MSCs, as well as the expression profile of epigenetic regulation enzymes (Diaz et al., 2015).

The present invention then relates to mesenchymal stem cells (MSCs) from fetal bovine bone marrow - an abundant source of MSCs, possessing broad differentiation potential and enabling the preparation of an allogeneic cell therapy composition - that is, of different individuals, and provide a method for therapy and its use, in the treatment of bovine mastitis.

The present invention provides experimental data for a method of treatment and use of fetal MSC of allogeneic origin for mastitis in milk producing animals. The composition can be administered intramammary, exerting an immunomodulatory effect on the inflammatory host response, strengthening the antimicrobial activity against the pathogens that cause mastitis and promoting the regeneration of the mammary glandular tissue, shortening the recovery time in dairy cows with mastitis and becoming an alternative for the replacement and / or complementation for the use of antibiotics.

## BRIEF DESCRIPTION OF THE INVENTION

An objective of the present invention is a composition of fetal mesenchymal stem cells (MSCs) of allogeneic origin for the treatment of mastitis in dairy animals, including bovines, and for immunostimulating said animal suffering from mastitis. In particular, MSCs come from fetal bovine bone marrow. The composition can be administered intramammary, exerting an immunomodulatory effect on the inflammatory response and strengthening the antimicrobial activity against pathogens that cause mastitis in milk-producing animals with mastitis and becoming an alternative for the replacement and / or supplementation for the use of antibiotics.

The present composition could be used in combination with an antibiotic selected from cephalosporins, penicillins, streptomycins, tetracyclines, or a combination thereof.

Another objective of the present invention is a method for treating mastitis in milk-producing animals, including bovines, and for immunostimulating said animal suffering from mastitis, which comprises administering fetal mesenchymal stem cells (MSCs) of allogeneic origin, said MSCs preferably coming from of fetal bovine adipose tissue, and preferably, said MSCs are administered intramammary.

It is still another objective for the present invention, the use of fetal mesenchymal stem cells (MSCs) of allogeneic origin in the treatment of mastitis in dairy animals, including bovines, and to immunostimulate said animal suffering from mastitis, said MSCs preferably coming from fetal bovine adipose tissue, and preferably, said MSCs are administered intramammary.

It is still another objective of the present invention, the use of fetal mesenchymal stem cells (MSC) of allogeneic origin in the preparation of a veterinary drug useful in the treatment of mastitis in dairy animals, including cattle, and to immunostimulate said animal that suffers from mastitis, said MSCs preferably coming from fetal bovine adipose tissue, and preferably, said MSCs are administered intramammary.

The present composition comprises a suspension of fetal bovine MSC of allogeneic origin that can be administered intramammary, and useful as an alternative or complementary treatment method to the use of antibiotics. The composition and form of intramammary administration allows the distribution of these cells in the mammary tissue, exerting an immunomodulatory effect on the inflammatory response, controlling the infectious process, and therefore, shortening the recovery time in dairy cows with induced mastitis.

**BRIEF DESCRIPTION OF THE FIGURES**

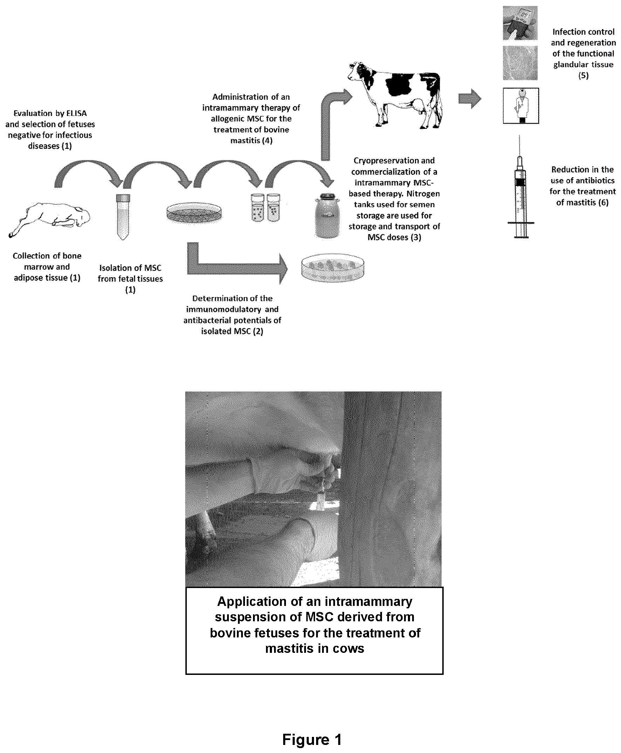

**Figure 1** Diagram of development and application of a treatment method for bovine mastitis based on allogeneic MSCs.

The diagram shows the isolation of MSCs from fetal tissues (1) to later characterize the proliferation and immunogenicity potential of MSCs and establish their potential treatment (2); the generation of a bank for cryopreservation that allows to have a permanent and immediate source of MSCs for its application and commercialization (3), and finally, the intramammary administration of MSCs would exert an immunomodulatory and regenerative effect of the glandular tissue that would reduce the use of antibiotics (4, 5 and 6).

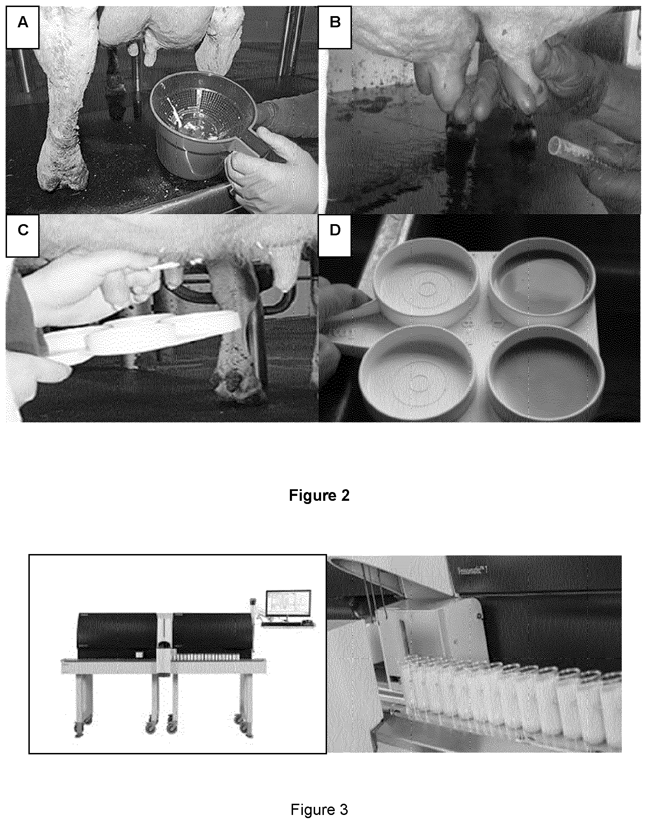

**Figure 2** Methodology for collection of milk samples in strip cup with dark surface, bacteriological and CMT evaluation in Holstein Friesian cows used in the study.

(A) The strip cup with dark surface test allows observing the presence of lumps in the first streams of milk projected on a container with a black background. (B) Each one of the nipples, starting with the most distant ones, was disinfected using a cotton swab with 70% alcohol. The previously labelled sample bottles were positioned at a 45 angle below the nipple without touching it to prevent contaminants from falling. Nearby nipples were first sampled and then distant nipples filling one third of the vial (C and D). The presence of subclinical and clinical mastitis was initially determined by CMT test (California Mastitis Test).

**Figure 3** Equipment Fossomatic FC 5000 (Foss, Denmark) based on flow cytometry technology was used for the count of somatic cells in milk samples from Holstein cows challenged with *S. aureus* and treated with fetal bovine MSCs.

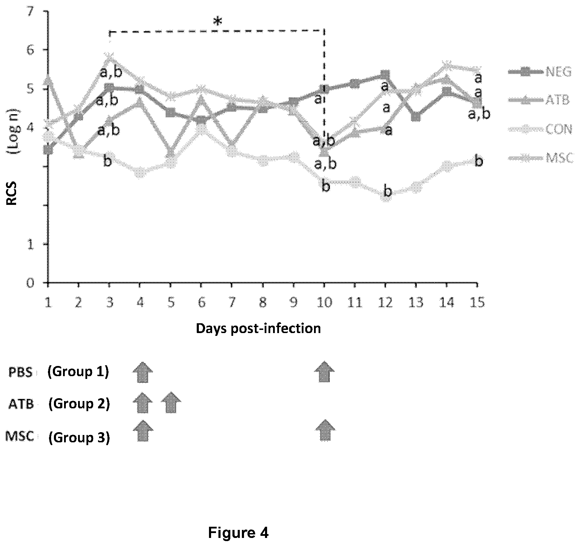

**Figure 4** Comparative analysis of SSC in cows challenged with *S. aureus* and treated with MSCs from fetal bovine origin. The left mammary quarters of each cow (n = 15) were challenged with *S. aureus* on day 1 and treated with Ringer Lactate, Group NEG (■), days 4 and 10 post-challenge), with antibiotics (Group ATB (▲), days 4 and 5 post-challenge), with MSC (MSC Group (✕), days 4 and 10 post-challenge). The right mammary quarters were inoculated with PBS (Phosphate Buffer saline) as a control for *S. aureus* (CON Group (●). The SSC were performed on milk samples obtained prior to milking. On day 10 post-challenge, a decrease ($P < 0.05$) in SCC was detected in group MSC compared to day 3 for the same group. Superscripts indicate significant differences ($P < 0.05$) between experimental groups (a, b) or between days post-challenge for a treatment (*). Abbreviation: CON, quarters not challenged with *S. aureus.*

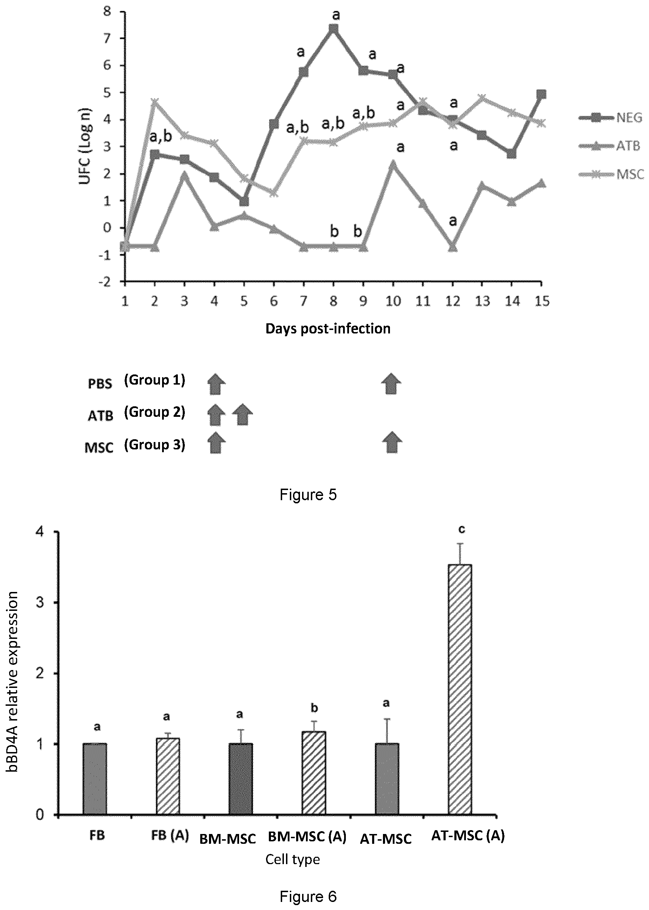

**Figure 5** Comparative analysis of CFU in cows challenged with *S. aureus* and treated with MSCs from fetal bovine origin. The left mammary quarters of each cow (n = 15) were challenged with S. aureus on day 1 and treated with Ringer Lactate, Group NEG (■), days 4 and 10 post-challenge), with antibiotics (Group ATB (▲), days 4 and 5 post-challenge), with MSC (MSC Group (✕), days 4 and 10 post-challenge). The CFU were determined in milk samples obtained prior to milking. The CFU values of the MSC group were intermediate (3.2, 3.2 and 3.8, respectively) and showed statistical difference compared to the NEG and ATB groups on days 7, 8 and 9 post challenge. Superscripts indicate significant differences (P<0.05) between experimental groups (a, b). Abbreviation: CON, quarters not challenged with S. aureus.

**Figure 6** mRNA levels of β-defensin 4A (bBD4A) in fetal bovine MSCs derived from bone marrow (BM-MSCs), adipose tissue (AT-MSCs) and in fetal fibroblasts (FBs) pre-exposed to *S. aureus.* The bBD4A mRNA levels increased in AT-MSC after activation (A) or exposure to S. aureus for a period of 6 hours. Different superscripts (a, b, c) indicate significant differences between treatments.

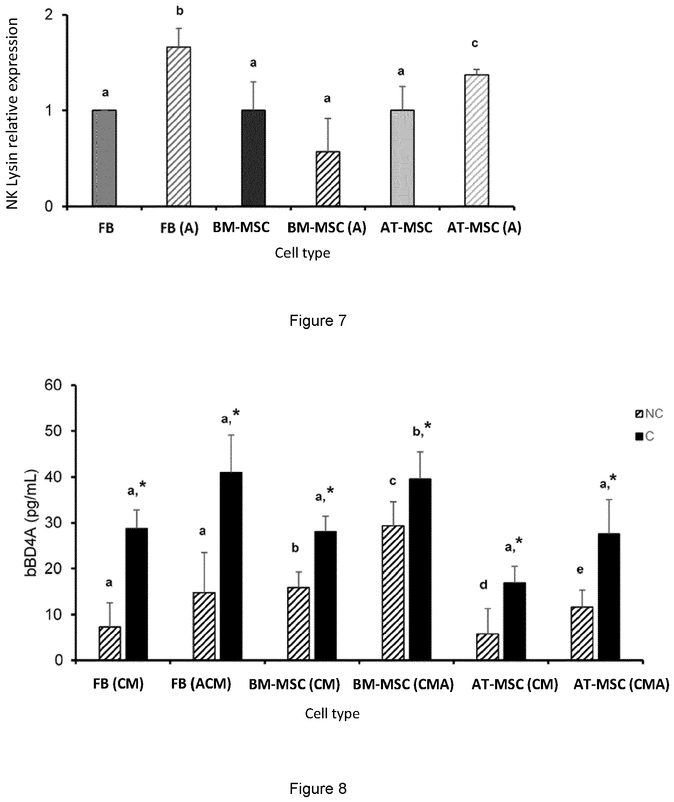

**Figure 7** NK-lysine 1 (NK1) mRNA levels in BM-MSCs, AT-MSCs and in FBs pre-exposed to *S. aureus.* NK1 mRNA levels increased in FBs and AT-MSCs after activation (A) or exposure to S. aureus for a period of 7 hours. Different superscripts (a, b, c) indicate significant differences between treatments.

**Figure 8** Expression of β-defensin 4A (bBD4A) in conditioned medium of fetal bovine BM-MSCs, AT-MSCs and of FBs pre-exposed to *S. aureus.* The expression levels of bBD4A increased (P <0.05) in BM-MSCs and AT-MSCs conditioned medium (CM) after activation (A) or exposure to S. aureus for a period of 6 hours. Additionally, the concentration of the conditioned medium by filtration increased (P <0.05) the levels of bBD4A in the conditioned medium. Different superscripts (a, b, c) indicate significant differences between cell types and activation treatment. Superscript (*) indicates significant differences between concentration treatment.

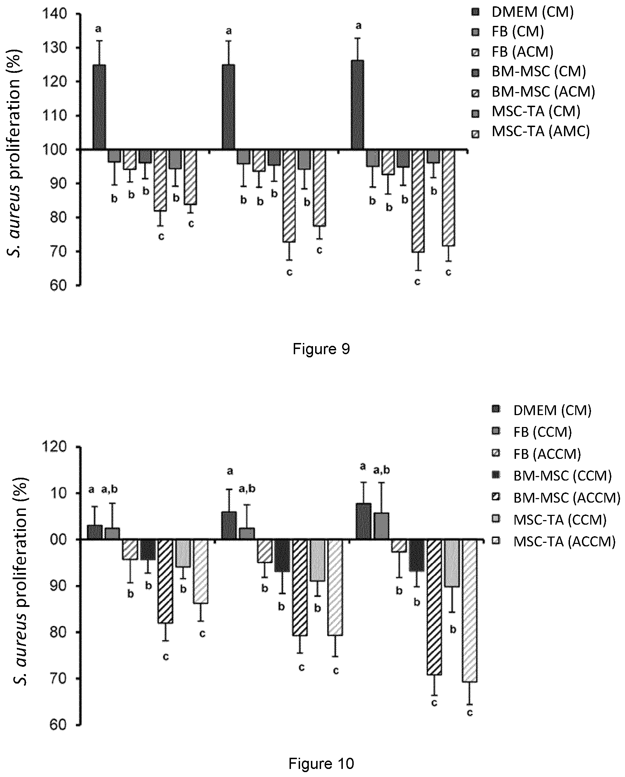

**Figure 9** Survival of *S. aureus* (SAU1 S) in activated conditioned medium (CM) from BM-MSCs, AT-MSCs and fetal bovine FBs as control. A decrease (P <0.05) was detected in the survival percentages of *S. aureus* exposed to activated conditioned medium of FBs, BM-MSCs and AT-MSCs compared to non-activated controls and control of DMEM medium alone. Different superscripts (a, b, c) indicate significant differences between treatments.

**Figure 10** Survival of *S. aureus* (SAU1 S) in concentrated activated conditioned medium from BM-MSCs, AT-MSCs and fetal bovine FBs as control. A decrease (P <0.05) was detected in the survival percentages of *S. aureus* exposed to activated conditioned and concentrated medium (ACCM) from BM-MSCs and MSC-TA compared to non-activated concentrated (CCM) controls and control of DMEM medium alone. Different superscripts (a, b, c) indicate significant differences between treatments.

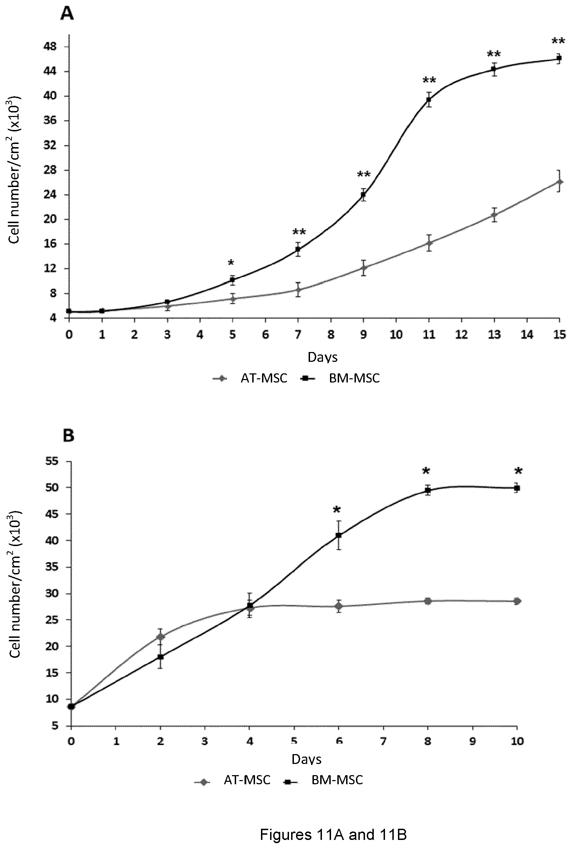

**Figures 11 A** and **11 B.** Proliferation curve of fetal bovine BM-MSCs and AT-MSCs with seeding concentrations of 5000 and 8500 cells/cm$^2$. A) With a seeding of 5000 cells / cm$^2$, the proliferation of BM-MSCs was higher (P <0.05) from day 5 to day 15 of culture compared to AT-MSCs. B) Using a seeding of 8500 cells / cm$^2$, a greater (P <0.05) proliferation of BM-MSCs was observed from day 6 of the culture compared to fetal bovine AT-MSCs. Superscripts (*, **) indicate significant differences (P <0.05 and P <0.01, respectively) between cell lines.

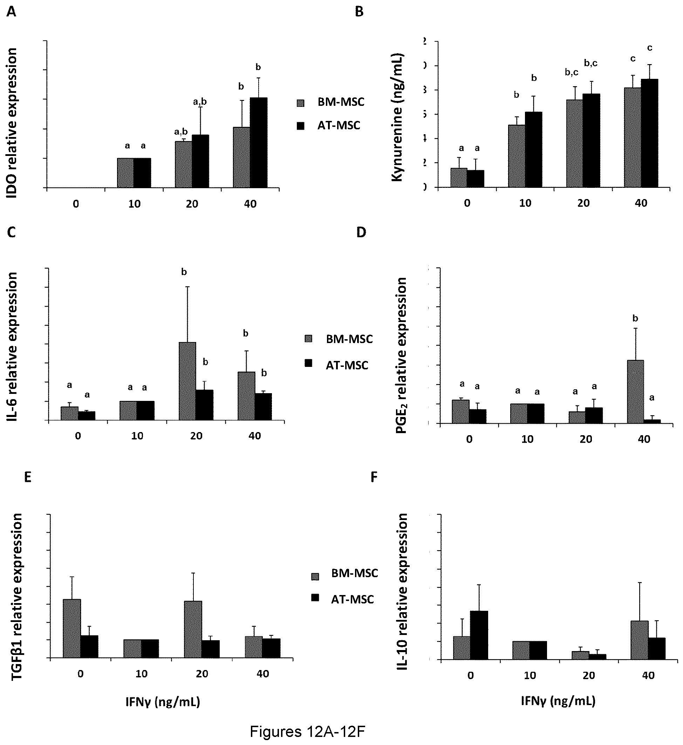

**Figures 12A-12F.** IDO mRNA levels, IL-6; PGE2, TGFβ1, IL-10 and kynurenine production in conditioned medium of BM-MSCs and AT-MSCs preactivated with IFNy. Figure 10 A BM-MSCs and AT-MSCs not treated with IFNy do not express detectable levels of IDO mRNA. However, MSCs treated with 40 ng/mL IFNy express higher (P <0.05) levels of mRNA compared to MSCs treated with 10 ng/mL IFNy. Figure 10B The kynurenine concentration in MSC conditioned media treated with 10 ng / mL IFNy were higher (P <0.05) compared to MSCs not treated with IFNy. In addition, higher (P <0.05) concentrations of kynurenine were detected in MSCs treated with 40 ng / mL IFNy compared to 10 ng / mL IFNy for both cell lines. Figure 10C BM-MSCs and AT-MSCs treated with 20 and 40 ng / mL IFNy expressed higher (P <0.05) levels of IL-6 mRNA compared to controls and MSCs not treated with IFNy. Figure 10D The levels of PGE2 mRNA in BM-MSCs preactivated with IFNy (40 ng / mL) were higher (P <0.05) compared to controls and AT-MSCs. The mRNA levels of TGFβ1 Figure 10E and IL-10 Figure 10F were similar for treatments and between cell lines. Abbreviation: IFNy, Interferon and; ND, means not detected; Different superscripts (a, b, c) indicate significant difference (P <0.05) between treatments.

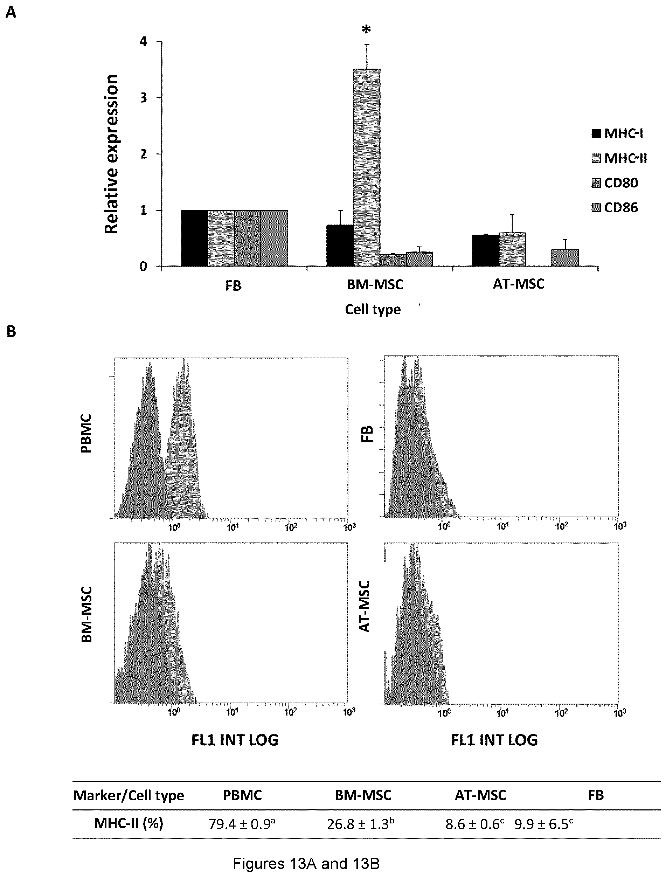

**Figure 13** Expression of major histocompatibility complexes (MHC) I and II, immunogenicity molecules, and mRNA levels of CD80 and CD86 co-stimulatory molecules in bovine fetal BM-MSCs and AT-MSCs. Figure 11A The mRNA expression levels of MHC-I, CD80 and CD86 were not different (P> 0.05) in the three cell lines. However, the relative expression of MHC-II in BM-MSCs was 3.51 times higher (P <0.05) compared to fibroblasts and AT-MSC (1 and 0.6 times, respectively). Figure 11B Analysis of MHC-II expression in bovine BM-MSCs, AT-MSCs, FBs and PBMCs by flow cytometry. A higher (P <0.001) proportion (79.4%) of PBMCs expressed MHC-II (green area) in relation to BM-MSCs (26,8%), AT-MSCs (8.6%) and FBs (9.9%). Likewise, a higher (P <0.001) proportion of BM-MSCs population was positive for MHC-II compared to AT-MSCs. Blue area, auto-fluorescence; Different superscripts (*, a, b, c) indicate significant differences (P <0.05), between cell lines.

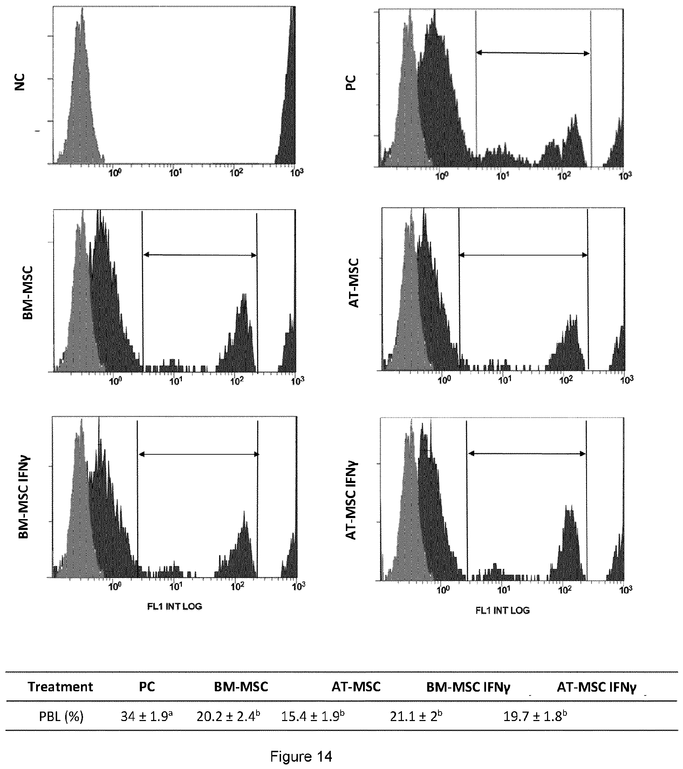

**Figure 14** Effect of conditioned medium of IFNy-preactivated BM-MSCs and AT-MSCs on the proliferation of CFSE-labelled and alloantigen-activated PBLs. The proliferation of bovine PBLs (between the first and second decade) in conditioned medium of both cell lines stimulated with or without IFNy was lower (P <0.01) compared to the positive control. Different superscripts (a, b) indicate a significant difference (P <0.01) between the positive control and the rest of the treatments. CN and CP: negative and positive control, BM-MSCs and AT-MSCs; MSC conditioned medium from bone marrow and adipose tissue, BM-MSC IFNy and AT-MSC IFNy; conditioned medium from IFNy-activated bone marrow and adipose tissue MSC.

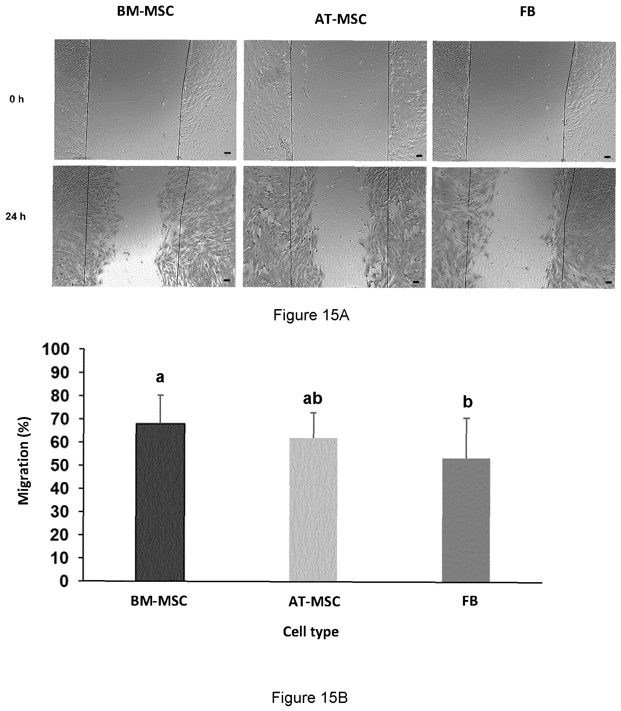

**Figure 15** In vitro migration potential of BM-MSCs, AT-MSCs and FBs evaluated by scratch assay. A. Photomicrographs of the different cell lines evaluated by the in vitro scratch test. B. Quantification of the in vitro migration potential of cell lines by scratch assay. BM-MSCs displayed a higher (P <0.05) migration value (68.1% $\pm$ 3.5) compared to FBs (53.6% $\pm$ 5.8) from bovine fetuses. Different superscripts (a, b) indicate significant differences p <0.05. Scale bar: 100$\mu$m.

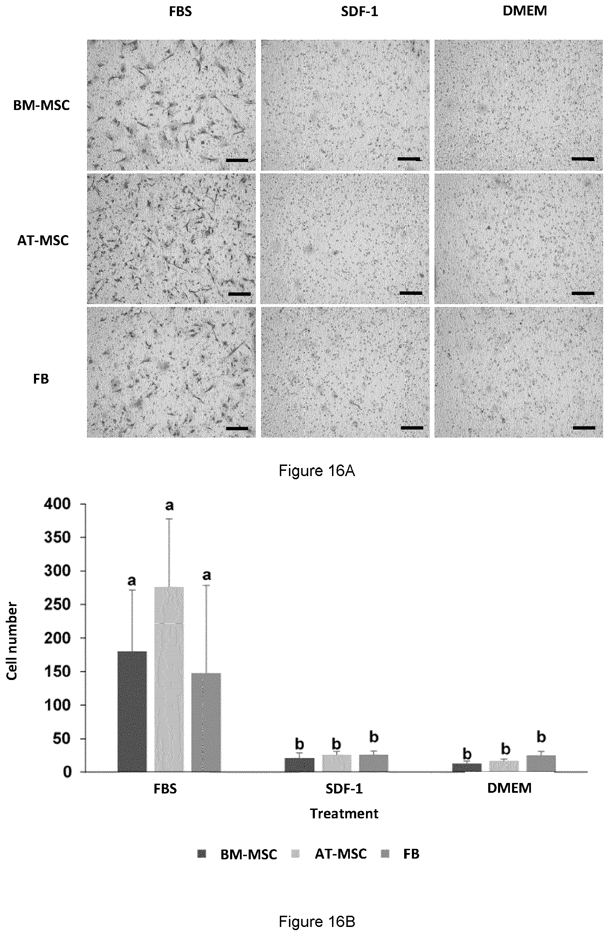

**Figures 16A** and **16B** Transwell analysis to determine the migration potential of BM-MSCs, AT-MSCs and FBs. A. Photomicrographs of migrants' cell populations in the transwell assay under the effect of SDF-1 (stromal cell-derived factor 1) and fetal bovine serum (FBS). B. Number of cells in migrant populations for each cell line under the effect of SDF-1 and 5% SFB. The highest (P <0.05) number of migrating cells was detected in cultures treated with DMEM supplemented with 5% FBS. Different superscripts (a, b) indicate significant differences p <0.05. Scale bar: 500 $\mu$m

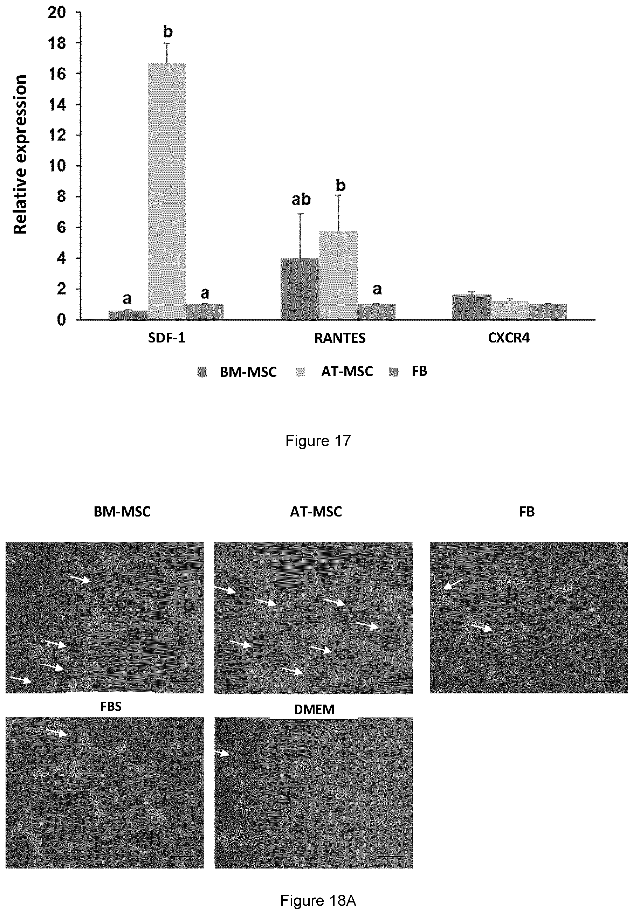

**Figure 17** Quantification of the mRNA levels of genes associated with migration in BM-MSCs, AT-MSCs and FBs. SDF-1 mRNA levels were higher (P <0.05) in AT-MSCs compared to BM-MSCs and FBs. RANTES mRNA levels were higher (P <0.05) in AT-MSCs compared to FBs. In the case of CXCR4, no differences in expression were detected between BM-MSCs, AT-MSCs and FBs. Different superscripts (a, b) indicate significant differences for each gene (p <0.05).

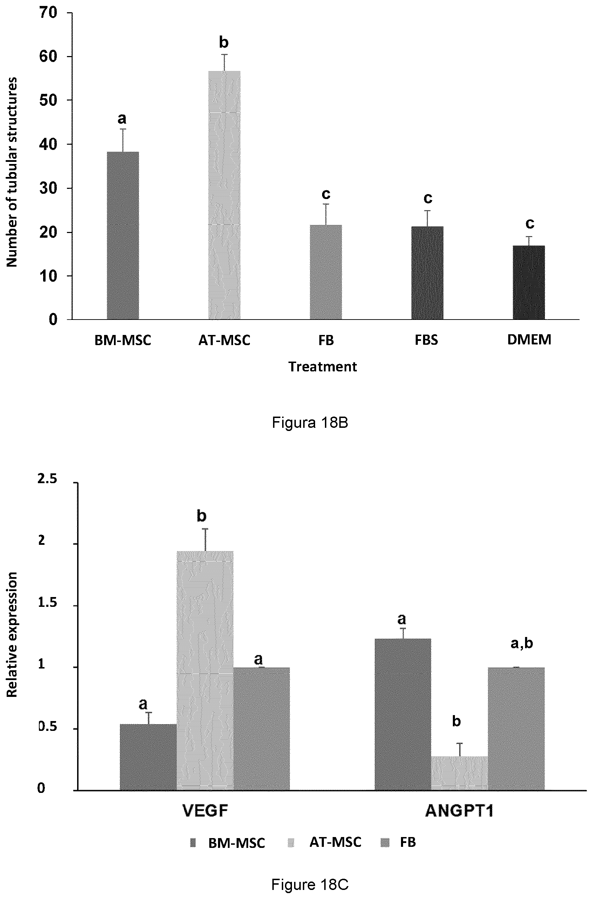

**Figures 18A and 18B** Angiogenic analysis by tubule formation assay in BM-MSCs, AT-MSCs and FBs. Figure 16A. Photomicrograph of the tubule formation assay of endothelial cells, exposed to concentrated conditioned medium from BM-MSCs, AT-MSCs for 6 hours. Figure 16B. Quantification of the number of tubules formed in endothelial cells, exposed to concentrated conditioned medium from BM-MSCs, AT-MSCs for 6 hours. The greatest (P<0.05) number of tubular structures was observed with conditioned medium from AT-MSCs. Regarding the conditioned medium from FBs, SFB and DMEM, their results do not difference (P>0.05) in terms of induction of tubular structure formation. Different superscripts (a, b, c) indicate significant differences p <0.05. Scale bar: 100$\mu$m

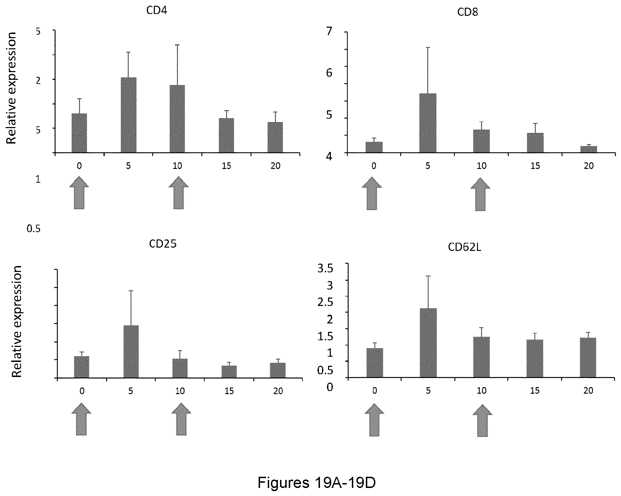

**Figure 19** Quantification of mRNA levels of genes associated with angiogenesis. AT-MSCs expressed higher (P <0.05) levels of VEGF mRNA compared to BM-MSCs and FBs (1.94 and 0.54 times the expression of FB respectively). In the case of ANGPT1, the relative expression of mRNA levels was higher in BM-MSCs and FBs compared to AT-MSCs (1.23 and 0.27 times the expression of FBs, respectively). Different superscripts (a, b, c) indicate significant differences for each gene (p <0.05).

**Figures 20A and 20B** mRNA levels of CD4, CD8, CD25 and CD62L lymphocyte activation markers in peripheral lymphocytes isolated from Holstein heifers inoculated with two doses of fetal bovine MSCs isolated from adipose tissue. The CD4, CD8, CD25 and CD62L mRNA levels were not different (P> 0.05) between sampling days. Arrows indicates day of inoculation of 25 x 10$^6$ MSC intramammary.

## DETAILED DESCRIPTION OF THE INVENTION

The present composition comprises a suspension of fetal bovine MSCs of allogeneic origin that can be administered intramammary; alternative or complementary treatment and use method to the use of antibiotics. The intramammary administration composition allows the distribution of these cells in the mammary tissue, exerting an immunomodulatory effect on the inflammatory response, controlling the infectious process, and therefore, shortening the recovery time in dairy cows with induced mastitis.

The present composition can be used in combination with an antibiotic selected from cephalosporins, penicillins, streptomycins, tetracyclines, or a combination thereof.

The present composition comprises fetal mesenchymal stem cells (MSCs) of allogeneic origin and serves in the treatment of mastitis in milk-producing animals, including bovines. The present composition is a substitute or complementary alternative to the use of antibiotics, increasing the effectiveness of the treatment and reducing the risk that its excessive use represents for public health.

The present regenerative mammary gland composition comprises allogeneic MSCs that are prepared by the following steps (Figure 1): Isolation of MSC from fetal tissues (1), in particular from adipose tissue. Determination of the potential for lymphocyte proliferation, immunomodulation and activation (2). Once isolated, the proliferation, immunomodulation, and lymphocyte activation potential of fetal MSCs is evaluated to confirm therapeutic properties.

Cryopreservation and commercialization of MSCs (3), the wide availability of tissues and therefore derived cells, allows the development of a pool of MSCs of allogeneic origin for cryopreservation with abundant reserves for their use and commercialization. Application of an intramammary treatment method for allogeneic fetal MSCs for mastitis (4 and 5). Consequently, there is a decrease in the use of antibiotics in order to treat mastitis (6).

To determine the in vivo effects of MSCs - which should be dependent on the population of stem cell origin used, BM-MSCs and AT-MSCs were compared in terms of proliferation, immunomodulation and lymphocyte activation potentials. The in vivo effects of MSCs were also dependent on the tissue into which they were inoculated. The potential for migration ("homing") and implantation ("engraftment") to the target tissue was evaluated by identifying fluorescently labeled MSCs in the parenchyma of the mammary gland.

The present invention relates to a suspension of mesenchymal stem cells (MSC) of fetal and allogeneic origin for the treatment of mastitis in dairy animals, including bovines.

The ability to migrate to the site of injury allows the treatment of mastitis, strengthening the immune response, exerting antimicrobial action and subsequently promoting the regeneration of damaged glandular tissue. The present composition also relates to a composition comprising such a suspension of allogeneic bovine MSCs derived from fetal tissues. This product can be stored by cryopreservation and can be administered intramammary.

The present invention would be a substitute or complementary alternative for the use of antibiotics, increasing the effectiveness of the treatment and reducing the risk that their excessive use represents for public health. Currently, there is no such tool available for the treatment of mastitis in dairy animals, including cattle, thus generating great therapeutic, productive and animal welfare benefits with the present invention.

Figure 1 shows a scheme for obtaining MSCs for the present invention. MSCs are first isolated from fetal tissues (1) from bone marrow or fetal adipose tissue. The use of fetal MSCs of allogeneic origin has various advantages over other sources of MSCs. First, the use of allogeneic sources makes it possible to have a permanent and immediate MSC bank, avoiding waiting time due to the need of cell extraction, isolation and expansion (4 to 6 weeks) - which differentiates from autologous therapies. Furthermore, the wide availability of tissues and therefore derived cells allows the development of a bank or storage of MSCs by cryopreservation. Second, the availability and differentiation capacity of MSC decreases during aging of individuals, so the use of fetal MSCs increases the potential efficacy of the solution proposed in this invention. Third, the use of allogeneic sources of MSC makes it essential to establish a microbiological control of infectious agents in donor individuals. For this, it is proposed to extract a blood sample from donor fetuses for the detection of Bovine Viral Diarrhea, Viral Rhinotrachitis, Enzootic Leukosis, Brucellosis and tuberculosis. The determination of the proliferation, immunomodulation and lymphocyte activation potentials of fetal MSCs may be performed. These analyses will make it possible to select the MSCs with the best therapeutic potential. In each case, it is important to highlight that MSCs obtained from fetal tissues have a greater capacity for lymphocyte proliferation and activation (2). Once isolated, the potential for proliferation, immunomodulation and lymphocyte activation of fetal MSCs was evaluated. These analyzes allowed evaluation with MSCs of high therapeutic potential. It is necessary to indicate that MSCs obtained from fetal tissues have a greater proliferation and differentiation capacity than MSCs obtained from adult tissues. In this sense, allogeneic therapy has the risk of inducing immune rejection in the host. However, fetal MSCs have low immunogenicity due to a lower expression of antigens of the Class I and Class II major histocompatibility complex, which makes them immune evasive cells. Cryopreservation and commercialization (3). The wide availability of tissues and, therefore, derived cells, allows the development of a bank of MSCs of allogenic origin for cryopreservation with abundant reserves for their use and commercialization. The use of allogeneic sources makes it possible to have this permanent and immediate "pool" of MSC. Importantly, MSC cryopreservation applies to procedures similar to those routinely used in commercial dairy for artificial insemination. This technical aspect includes the similarity both in cell packaging and in storage and thawing temperatures, which will facilitate the use and application of MSC therapy for the treatment of mastitis (4 and 5). The intramammary route (nipple canal) allows the application of this therapy to be similar to the intramammary antibiotic therapies currently used. This represents a great application advantage from a practical point of view since the dairy producer knows this type of procedure. The recognized migration ability of MSCs is distributed in damaged glandular tissue after intramammary inoculation. The immunomodulatory capacity of MSCs will allow reducing treatment time with MSC alone or in combination with antibiotic therapy. Additionally, due to their ability to release cellular factors, MSCs can generate an important antimicrobial contribution and in the regeneration of the histoarchitecture of the mammary gland. Reduce in the use of antibiotics for the treatment of mastitis (6).

Trials were conducted to evaluate efficacy parameters of intramammary administration of the allogeneic composition of fetal bovine MSC in cows with induced mastitis.

In the examples below - unless otherwise stated, trials were conducted using 15 clinically healthy (free from Brucellosis, Tuberculosis and Leukosis) Holstein Friesian cows of approximately 2 years of age. Three bacterial milk cultures were performed to rule out the presence of intramammary infections. Cows were milked twice daily using a separate milking unit and milk production was quantified after each milking. The trial was conducted according to the Applicant's Bioethics guidelines. The animals were kept under the same management and were fed alfalfa and concentrate.

Example 1: Preparation and administration of the inoculum

A strain of *S. aureus* was thawed and seeded on blood agar and incubated at 37 °C in aerobiosis overnight. A bacterial colony of S. aureus was then incubated in 5 mL of brain heart broth in a 15 mL tube for 10 hours at 37 °C. Subsequently, eight dilutions of the broth were prepared in PBS and 100 uL of each dilution were seeded in duplicate blood agar. Using a sterile rake, the inoculum of each dilution was distributed on the surface of each plate. The plates were incubated overnight in aerobiosis at 37 °C. Subsequently, the colonies on the plate containing between 30-300 CFU were counted in duplicate and averages were obtained. To calculate the number of CFU per mL, the equation (Eq. 1) was used:

$$\text{Number of colonies} \times (10)^A \ (\text{number of the dilution} + 1) = \text{Amount of CFU / mL (Eq. 1)}$$

The volume necessary to obtain a bacterial suspension with a concentration of 1000 CFU / mL in 1 Lt of PBS was calculated using the equation (Eq. 2):

$$C1 \times V1 = C2 \times V2 \ (\text{Eq. 2})$$

where C1 = Concentration expressed in CFU / mL, V1 = Volume to be extracted from the incubated broth, C2 = Desired concentration (1000 CFU / mL) and V2 = Total volume of the suspension for inoculate (1000 mL).

Example 2: Challenge

The cows (n = 15) at approximately 30 days postpartum were challenged by inoculation of the two left mammary quarters via the intracisternal route administration of 1 mL of a 1000 cfu / mL suspension of S. aureus. 1 mL of PBS was inoculated into the right quarters as a control.

From the day of the challenge and daily for 15 days, the cows were clinically evaluated and milk samples were collected to determine SCC and CFU. The clinical evaluation and the collection of milk samples were carried out as detailed below.

*Experimental design*

The cows were randomly assigned to three experimental groups of 5 animals each. All the animals were challenged by inoculation of the two left mammary quarters via the intracisternal route using 1 mL of a 1000 CFU / mL suspension of S. aureus.

1 mL of PBS was inoculated into the right quarters as a control. The NEG group (without therapy) was treated on days 4 and 10 with a 5 mL Ringer Lactate solution via the teat canal in the two infected quarters. The ATB group (antibiotic therapy) was treated with a commercial suspension of 50 mg of pirlimycin on experimental days 4 and 5 via the teat canal in the two infected quarters. The MSC group (MSC therapy) was administered with a 2.5 x 10[7] suspension of MSC in 3 ml_ of Lactated Ringer via the teat canal in the two infected quarters. From the day of the challenge and daily during 15 days, the cows were clinically evaluated and milk samples were collected daily to determine SCC and CFU (Figure 2B). The clinical evaluation and the collection of milk samples were carried out as detailed below.

*Clinical examination*

From the day of the challenge and daily during 15 days, the cows were evaluated by clinical examination that included determination of heart rate, respiratory rate, ruminal rate, rectal temperature, and mucous coloration. According to the classification table provided below (Table 1.; Wenz J, Garry F, Barrington G. 2006. Comparison of disease severity scoring systems for dairy cattle with acute coliform mastitis. JAVMA. 229, 259-262). From two days prior to the challenge and daily, the udder of each cow was evaluated after each milking by manual palpation of each mammary quarter to detect abnormalities such as volume increase, pain, redness, and temperature increase (Table 1). The abnormalities in the appearance of the milk were evaluated at the beginning of milking using a strip cup with dark surface to classify the secretion as normal, watery, viscous, with the presence of lumps, blood or pus (Figure 2A). The presence of subclinical and clinical mastitis was initially determined using the CMT test (California Mastitis Test) (Figure 2C and D). The CMT test consists of a subjective density and colorimetric reaction in milk because of the reactivity of deoxyribonucleic acid (DNA) of somatic cells in milk with the CMT reagent. A sample of milk obtained from each mammary quarter is mixed with a similar amount of CMT reagent in each of the wells of the paddle. The reaction has a scale of 0 to 3 in which the interval 0 to 1 is negative and the interval 2 to 3 is positive and represents an SCC greater than $3 \times 10^5$. The evaluation of the severity of the clinical signs of the udder was carried out according to a previously reported score scale (Wenz J, Garry F, Barrington G. 2006. Comparison of disease severity scoring systems for dairy cattle with acute coliform mastitis. JAVMA. 229, 259-262; Table 2). This classification includes the following variables, firmness of the quarter (not firm, firm or very firm), increase in volume of the quarter (no increase in volume, 1.5 times greater or 2 times greater), pain in the quarter (no pain or with pain) and discharge characteristics (normal, thin with clots or serum-like with clots).

**Table 1**.Dairy cow clinical signs severity classification system based on systemic signs of the disease.

| Variable | Criteria | Score |
|---|---|---|
| Rectal temperature (°C) | 37.8 - 39.27 | 0 |
| | 39.33 - 39.8 | 1 |
| | >39.8 o <37.8 | 2 |
| Hydratation status (degree of enophthalmos) | None | 0 |
| | Mild | 1 |
| | Moderate | 2 |
| | Marked | 3 |
| Rumen contraction rate (contractions/min) | $\geq 2$ | 0 |
| | 1 | 1 |
| | 0 | 2 |
| Attitude (signs of depression) | None | 0 |
| | Mild | 1 |
| | Marked | 2 |
| Cows with total score of 0 to 2 were classified as having mild disease, cows with total score of 3 to 5 were classified as having moderate disease, and cows with total score of 6 to 9 were classified as having severe disease. | | |

**Table 2.** Classification system for the severity of clinical signs in the udder of dairy cows based on local signs of the disease.

| Variable | Criteria | Score |
|---|---|---|
| Quarter firmness | None | 0 |
| | Firm | 1 |
| | Very Firm | 2 |
| Quarter swelling | None | 0 |
| | 1.5 times | 1 |
| | 2 times | 2 |
| Signs of quarter pain | None | 0 |
| | Present | 1 |
| Secretion characteristics | Normal | 0 |
| | Thin with clots and flakes | 1 |
| | Serum-like with clots or flakes | 2 |
| Cows with total score of 0 to 2 were classified as having mild disease, cows with total score of 3 or 4 were classified as having moderate disease, and cows with total score of 5 to 7 were classified as having severe disease. | | |

*Bacteriological analysis (CFU)*

Milk samples prior to milking were collected aseptically from all cows following the protocols of the International Dairy Federation (1985) on three consecutive days prior to the experimental challenge (Figure 2B). Briefly, before milking, the quarters and teats were cleaned using a brush or washed with water in case of excessive presence of mud or manure. Then the teats and especially the tips of the teats were disinfected using paper towels with 70% alcohol. Three or four milk streams were discarded to reduce the possibility of contamination with pathogens present in the teat canal. Each of the teats, starting with the most distant ones, were disinfected again using a 70% alcohol swab. Previously labelled sample bottles were positioned at a 45-degree angle below the nipple without touching it to prevent contamination. Nearby teats were first sampled and then distant teats, filling one third of the vial. The samples were refrigerated or frozen for analysis of SCC or CFU, respectively. Only cows with three negative consecutive cultures were used in the study. After challenge, milk samples prior to daily milking were collected aseptically from all cows for isolation of S. aureus during the 15 days of the study. Bacteriological analyses were performed according to standards accepted and recommended by the National Mastitis Council, USA (Hogan SJ, Gonzalez NR, Harmon JR, Nickerson CS, Oliver PS, Pankey JW, Smith KL. 1999. Laboratory Handbook on 10 Bovine Mastitis. Rev ed, National Mastitis Council Inc, Madison, WI, USA. pp 222). The separated samples were cultured on blood and MacConkey agar anaerobically at 37 °C for up to 48 h. The types of bacteria were characterized according to their Gram stain reaction, hemolytic properties and morphology of the colonies. Staphylococcus isolates were identified as S. aureus and Staphylococcus Coagulase Negative (SCN) using catalase, tube coagulase, and fermentation assays for the production of glucose acid, mannitol, and maltose. Escherichia coli and other Enterobacteriaceae were identified by catalase, oxidase and IMVIC assays (Indole, Methyl Red, Voges proskeur, and citrate) and on MacConkey agar and EMB. Gram negative identification was carried out using a strain identification system (Microbacter GNB 24E, Oxoid UK). Streptococci spp was identified using CAMP test, esculin hydrolysis, hemolysis, and lack of growth on MacConkey agar. The presumptive S. aureus colonies based on their morphology were subcultured on a new blood agar plate and tested for coagulase production by means of a test tube with rabbit plasma with EDTA (Ethylenediaminetetraacetic acid). Staphytect Plus rapid kit was used for the identification of S. aureus.

*Somatic cell count (SCC)*

From two days prior to the challenge and daily for the 15 days of the study, milk samples were collected prior to milking from all cows in 50 ml plastic tubes with bronopol as preservative. SCC was performed on the milk samples using a Fossomatic flow cytometer (Figure 3).

*Obtaining and cultivating MSC*

MSCs were isolated from bovine fetal adipose tissue. Bovine fetuses at the last third of gestation were obtained from an abattoir and transported in a thermal container to the laboratory. Fetal adipose tissue was extracted by incision in the abdominal midline and surgical extraction using a scalpel and sterile forceps. Then the adipose tissue was washed with phosphate buffer solution (PBS), pH 7.4 and sectioned using a scalpel. Subsequently, the tissue was incubated at 38 °C for 45 min under constant agitation in a digestion medium consisting of 0.5% collagenase diluted in HBSS solution (Hank's Balanced Salt Solution). The enzymatic reaction was stopped by adding expansion medium consisting of DMEM medium (Dulbecco's Modified Eagle's Medium) supplemented with 10% fetal bovine serum, 500 uL of amphotericin B and 500 uL of penicillin / streptomycin in an amount equivalent to the digestion medium. Subsequently, the tissue was filtered using pores of 40 um and centrifuged twice at 400 xg for 5 min using washing medium, which consisted of DMEM supplemented with 100 ug / mL of amphoterecin B, 100 IU / mL of penicillin and 100 ug / mL streptomycin. The pellet was resuspended in expansion medium, and then transferred to 175 cm$^2$ cell culture flasks. MSC cultures derived from adipose tissue were incubated at 38 °C under a humid atmosphere with 5% $CO_2$. After 48 hours, the cells not adherent to the plastic were removed by changing the culture medium. Upon reaching 80 to 90% confluence, MSC passages were performed by removal with trypsin / EDTA, supplemented with 250 u.g / mL of amphoterecin B, 100 IU / mL of penicillin and 100 u.g / mL of streptomycin.

*Statistical analysis*

The data on clinical variables, SSC and CFU were analysed using the Shapiro Wilks normality test. Treatment effects were evaluated by Kruskal Wallis analysis. The experimental days and the treatment were evaluated as independent variables, and the clinical variables, the SCR and the CFU were evaluated as dependent variables. A significance value ($P < 0.05$) was used. All statistical analyzes were performed using the Info Stat software (Córdoba, Argentina, 2008).

*Comparative analysis of clinical variables in cows challenged with S. aureus*

The cows showed an increase in volume of the challenged quarters from day 2 post-challenge. No traces of blood or pus were observed in the milk by strip cup with dark surface testing but lumps were detected in samples from all cows intermittently during the study. The average values of the clinical variables evaluated including heart rate, respiratory rate, ruminal rate, rectal temperature, thermography of the mammary quarters, and milk production did not show differences (P> 0.05) between the experimental groups (Table 3). The average daily milk production of the NEG, ATB and MSC groups was 17.3 ± 1.8; 16.7 ± 0.8 and 14.7 ± 1 l, respectively. There was a decrease in milk production between days 5 and 7 of the study of 6.8; 2.1 and 2.3 (NEG, ATB and MSC Groups, respectively).

**Table 3.** Clinical and productive variables in cows (n = 5 each group) challenged with S. aureus and assigned to experimental groups NEG (no therapy), ATB (antibiotic therapy) and MSC (therapy with MSC).

| Variable/Treatment group | NEG | ATB | MSC |
|---|---|---|---|
| Heart rate (bpm) | 59.2 ± 3.8 | 53.3 ± 3,5 | 53.1 ± 1.6 |
| Respiratory rate (bpm) | 30.9 ± 3.4 | 28.6 ± 2.5 | 30.5 ± 1.9 |
| Ruminal frequency (in 2 min) | 2.9 ± 0.3 | 2.6 ± 0.3 | 2.4 ± 0.2 |
| Rectal temperature (°C) | 38 ± 0.7 | 38.1 ± 0.3 | 37.9 ± 0.2 |
| Termography FL (°C) | 34.1 ± 0.8 | 33.8 ± 0.7 | 34.4 ± 0.7 |
| Termography BL (°C) | 34.2 ± 2.5 | 34.3 ± 0.6 | 34 ± 0.6 |
| Termography FR(°C) | 33.8 ± 0.9 | 33.7 ± 0.7 | 33.6 ± 0.5 |
| Termography BR (°C) | 34.1 ± 1.2 | 34.2 ± 0.5 | 34 ± 0.4 |
| Milk yield (lts.) | 17.3 ± 1.8 | 16.7 ± 0.8 | 14.7 ± 1 |
| Milk yield reduction days 5-7 (lts) | 6.8 | 2.1 | 2.3 |
| Values represents means ± standard deviations | | | |

*Comparative analysis of RCS in cows challenged with S. aureus*

From the day of the challenge and daily for 15 days, milk samples were collected to determine SCC. The SCC values were subsequently expressed as a linear score (LS-SCC) by means of logarithmic transformation (LS = LOGN). The mammary quarters of the NEG and ATB groups (without therapy and antibiotic therapy, respectively) obtained statistically similar LS-SCC values compared to the uninfected quarters from day 1 to day 10 post-challenge (Figure 4). The mammary quarters of the MSC group (MSC therapy) obtained higher LS-SCC values (P <0.05) compared to the non-infected quarters on day 3 post-challenge (MSC group = 7 vs. non-infected quarters = 3, 2). The LS-SCC values of the MSC group were not different (P> 0.05) compared to the NEG and ATB groups during the 15 days of the study. However, on day 10 post-challenge, a decrease (P <0.05) of LS-SCC was detected in the MSC group compared to day 3 for the same group (3.6 vs. 7). The NEG, ATB and MSC groups obtained similar PL-RCS values (P> 0.05) from day 10 to day 15, which were different from the non-infected rooms on days 12, 13 and 14 post-challenge.

*Comparative analysis of CFU in cows challenged with S. aureus*

From the day of the challenge and daily for 15 days, milk samples were collected for determination of CFU. The CFU values were subsequently expressed as a linear score (LS-CFU) by means of logarithmic transformation (LS = LOGN). Milk samples from the MSC group reached higher (P <0.05) PL-CFU (4.6) values compared with the samples obtained from the ATB group quarters (-0.7) on day 3 post-challenge (Figure 5). On this day, the LS-CFU values of the milk samples from the NEG group were not different (P> 0.05) compared to the milk samples obtained from the quarters of the ATB and MSC groups. The LS-CFU values of the NEG group increased (P <0.05) on days 7, 8 and 9 (5.8, 7.4 and 5.8, respectively) compared to the quarters of the ATB group (-0, 7, -0.7 and -0.7, respectively). The LS-CFU values of the MSC group were intermediate (3.2, 3.2 and 3.8, respectively) and did not show statistical difference compared to the NEG and ATB groups on days 7, 8 and 9 post-challenge. The LS-CFU values of each group were not different (P <0.05) from day 13 to day 15 of the study.

The previous example demonstrates that the cows challenged with S. aureus and assigned to the MSC group reached the highest values of SCC and CFU (Days 2 and 3, respectively), after the challenge with S. aureus but prior to the administration of MSC. However, the group of cows treated with MSC significantly decreased SCC values on day 10 post-challenge. Furthermore, the group of cows treated with MSC reached intermediate values of CFU without showing significant differences compared to the groups without therapy and with antibiotic therapy. These data confirm that MSCs exerted an immunomodulatory and antibacterial effect in cows challenged with S. aureus.

*Example 3 Antibacterial Potential of MSCs derived from bone marrow and fetal bovine adipose tissue*

Strains ATCC 25923 and SAU-1 S of S. aureus were used in order to determine their survival in unconcentrated MSC conditioned medium (CM), concentrated MSC conditioned medium (CCM), MSC conditioned medium activated by pre-exposure of S. aureus (ACM). In these same MSC cultures, the mRNA levels of the antimicrobial peptides b-defensin 4A (bBD4A) (Figure 6) and NK-lysine 1 (NK1) (Figure 7) were quantified by Q-PCR and of bBD-4A by Elisa (Figure 8).

The CM were obtained from BM-MSCs, AT-MSCs and FBs cultures as control. Cells were seeded at a concentration of $6 \times 10^3$ cells / $cm^2$ until reaching 70 to 80% of confluency. Subsequently, MSCs and FBs were cultured in DMEM medium with 4500 mg / L D-Glucose (Mediatech, Incorporated) without serum, antibiotic and antifungal for 72 h at 38 °C under a humid atmosphere with 5% $CO_2$. Next, the CM were collected and then centrifuged at 400 x g for 5 min and later filtered through pores of 0.22 $\mu$m and frozen at -20 °C until use. For the experiments with concentrated CM, the medium was concentrated 10 times its initial volume, by ultrafiltration using Amicon Ultra filters (Merck Millipore, Corporation, Cork, Ireland) with a 3 kDa NMWL membrane according to the manufacturer's instructions. The concentrated media were then frozen at -20 °C until used. Subsequently, the antibacterial potential of the activated CM obtained from cultures of BM-MSC, AT-MSCs, as well as FBs that were previously exposed to 300 CFU of the local strain SAU-1 S of S. aureus by a 6 h period (Figure 9). In this test, a decrease (P <0.05) in the survival of S. aureus was detected at 1, 2 and 3 hours of post-bacterial inoculation culture in the activated conditioned media of BM-MSCs (81, 95; 72 , 86 and 79.85%, respectively) and AT-MSCs (83.92, 77.48 and 71, 62%, respectively) compared to the DMEM control medium (124.89, 124.96 and 126.20% , respectively). Likewise, these survival values of S. aureus were lower (P <0.05) compared to the non-activated conditioned media of BM-MSCs (96.14, 95.44 and 94.92%, respectively), of AT-MSCs (94.38, 94.22 and 96.10%, respectively), of activated FBs (94.14, 93.62 and 92.64%, respectively) and of non-activated FBs (96.35, 95, 84 and 95.06%, respectively). However, no differences (P> 0.05) were found in survival levels of S. aureus between BM-MSCs and AT-MSCs activated CM. Likewise, the survival percentages were not different between the incubation hours. Additionally, the antibacterial potential of the activated and concentrated CM that was obtained from BM-MSCs and AT-MSC cultures, as well as FB cultures that were previously exposed to 300 CFU of the local strain SAU-1 S of S. aureus for a period of 6 h, which was subsequently concentrated (10 times) (Figure 10). In this test, a decrease (P <0.05) in the survival of S. aureus was detected at 1, 2 and 3 hours of post bacterial inoculation culture in the activated CM and concentrated BM-MSCs (82.01; 79.28 and 70.86%, respectively) and AT-MSCs (86.21; 79.31 and 69.27%, respectively) compared to the DMEM control medium (103.06, 105.97 and 107.71%, respectively). Likewise, these survival values of S. aureus were lower (P <0.05) compared to the concentrated non-activated CM of BM-MSCs (95.70, 93.1 1 and 93.23%, respectively), of AT-MSCs (94.07, 91, 03 and 89.86%, respectively), of activated FB (95.72, 95.05 and 97.31%, respectively) and of non-activated FB (102.41 ; 102.40 and 105.70%, respectively). However, no differences (P> 0.05) were found in survival rates of S. aureus between the activated and concentrated CM of BM-MSCs and AT-MSCs. Likewise, the survival percentages were not different between the incubation times.

Consequently, the above tests indicate that BM-MSCs and AT-MSC CM possess antibacterial effect against S. aureus in an in vitro culture system. This antibacterial potential is increased by concentrating the media by filtration and activating the CM by pre-exposure of MSCs to S. aureus. The results suggest that the antibacterial effect of MSCs is mediated by the expression of antimicrobial peptides NK1 and bBD4A. See Figures 6 and 7.

*Example 4 Determination of the proliferation potential of fetal MSCs derived from bone marrow and bovine adipose tissue*

Isolation of MSC from fetal bone marrow (BM-MSCs). Bone marrow was obtained from femurs of bovine fetuses of 8 to 9 months of gestation (n = 5) derived from slaughterhouse (Figure 1). The donor fetuses were sampled to rule out the presence of Viral Diarrhea, Viral Rhinotracheitis, Enzootic Leukosis, Brucellosis and Tuberculosis. The bone marrow samples were aspirated through an 18 G cannula connected to a 10 mL syringe with 2 ml of collection medium containing DMEM; supplemented with 1000 IU / mL of Heparin, 100 $\mu$g / mL of Streptomycin and 100 $\mu$g / ml of Penicillin. The aspirate was centrifuged twice in a phosphate buffer solution, pH 7.4 and twice in a collection medium without heparin at 300 G for 5 minutes at room temperature (RT). The pellet was aspirated and resuspended in expansion medium, which consisted of high glucose DMEM (4500 mg/ L D-Glucose) supplemented with 10% FBS, 100 $\mu$g / mL of Streptomycin and 100 $\mu$g/ml of Penicillin in a Petri dish and incubated at 38 °C under a humid atmosphere with 5% $CO_2$. After two days of culture, cells non-adherent to the plastic were removed by washing with PBS and changing the culture medium.Isolation of MSC from adipose tissue (AT-MSCs). The adipose tissue was obtained from the peritoneum of bovine fetuses of 8 to 9 months of gestation (n = 5). The samples were washed three times with collection medium. The supernatant was removed and mechanical disintegration was performed. Then, the extracellular matrix was digested with 0.1% Collagenase II at 38 °C under stirring. Collagenase was inactivated 12 h later by adding a volume of expansion medium. The suspension obtained was centrifuged at 400 G for 10 minutes at RT. Subsequently, the BM-MSC-MOs and AT-MSCs were seeded separately in expansion medium consisting of DMEM supplemented with 5% fetal bovine serum (FBS). Upon reaching 80 to 90% cell confluence, passages were made by stirring with 0.25% Trypsin / EDTA for 5 min. The MSCs were cultivated in expansion medium until reaching 80% confluence.

The proliferation potentials of bovine BM-MSCs and AT-MSC were compared by calculating population doubling (DP) according to tissue of origin. Population doublings were determined. BM-MSCs and AT-MSCs were seeded separately in triplicate in 3.5 cm culture plates in expansion medium until reaching 70 to 80% confluence. Then the MSCs were passed at a concentration of $8.4 \times 10^3$ cells / $cm^2$. Cells from passages 1 to 15 were maintained with a fixed subculture period of 5 days and counted and reseeded. The cell count data were used to estimate the population doubling and to construct an accumulated population doubling by means of the equation (Eq. 1):

$$DP = \ln (Nf / Ns) \ (Eq. \ 1)$$

Where ln is the natural logarithm, Nf is the number of cells obtained in the subculture and Ns is the number of cells initially seeded (Freshney RI. 2005. Culture of Animal Cells: A Manual of Basic Technique. 5th edition. Editorial Wiley-Liss. New Jersey. 38-358). To establish a proliferation curve with the total cumulative number of cells during the passages, the equation (Eq. 2) was used:

$$\text{Number of total cells of } P_n = \text{Number counted in } P_n \times (\text{Number counted at Pn-i / Total}$$

$$\text{number of cells seeded } ) \ (Eq. \ 2)$$

The proliferation potential was determined at two concentrations (5000 and 8500 cells / $cm^2$) for 15 days (Figure 11 A) and 10 days (Figure 11 B) of culture, respectively.

The proliferation and immunogenicity potentials of MSCs are dependent on the source of the tissue of origin, since MSCs derived from bone marrow possess high proliferation and immunogenicity potentials compared to MSCs derived from adipose tissue. BM-MSCs have greater proliferation potential, due to the fact that they double their population in less time compared to AT-MSCs from bovine fetuses. However, both BM-MSCs and AT-MSCs possess similar immunomodulation potentials.

*Example 5 Determination of the immunomodulatory potential of BM-MSCs and AT-MSCs by gene expression analysis of paracrine immunomodulatory factors (IDO, IL-6, IL-10, PGE$_2$ and TGFβ1) and determination of the enzymatic activity of IDO in conditioned medium of MSC preactivated with IFNy*

mRNA levels of paracrine immunomodulatory factors in BM-MSCs and AT-MSCs preactivated with IFNy. The immunomodulatory potential was evaluated by quantifying the expression of anti-inflammatory genes (IDO, IL-4, IL-10) in BM-MSCs and AT-MSCs treated with 10, 20 or 40 ng/mL of IFNy. (Figure 12). The MSC samples at the second passage were fixed as previously described and the expression of these genes was analyzed by quantitative PCR (Q-PCR). RNA extraction and cDNA synthesis. Total RNA was extracted from the cells, using an RNAeasy kit (Qiagen), following the manufacturer's instructions. Total RNA was quantified using a Qubit RNA HS kit (Life Technologies).

For the reverse transcription (RT) reaction, a Brilliant II SYBR Green RT-PCR kit (Agilent Technologies) was used. The reaction was carried out according to the manufacturer's instructions. Q-PCR. Amplification reactions were performed using a Brilliant SYBR Green Q-PCR Master mix kit (Stratagene). Each reaction tube contained 12.5 µL of Master mix, 2.5 µL of forward primer, 2.5 µL of reverse primer (Integrated DNA Technologies, Inc., Illinois, USA), 0.375 µL of Rox reference stain, 2 µL of cDNA and $H_2O$ until reaching a total volume of 25 µL. The theoretical denaturation temperature (Tm) analysis was carried out after amplification through a cycle that consisted of increasing the temperature from 95 °C at a rate of 20 °C / s for the total denaturation of the double strand, then lowering the temperature at 65 °C at a rate of 20 °C / s for renaturation and finally raising to 95 °C at a rate of 0.1 C / s, recording the loss of fluorescence every 0.1 °C. To ensure that the amplicons were generated from mRNA and not from genomic DNA amplification, controls without reverse transcriptase were included.

The Q-PCR data obtained were analyzed using the comparative method of Ct, in relation to the Ct of the endogenous genes GAPDH and p-ACTIN (Schmittgen TD, Livak KJ. 2008. Analyzing real-time PCR data by the comparative C (T) method. Nat Protoc 3, 1 101 -1 108). Determination of the enzymatic activity of IDO in conditioned medium of MSCs preactivated with IFNy. BM-MSCs and AT-MSCs from 2nd to 3rd passage were seeded at a concentration of $1 \times 10^4$ cells / cm$^2$ in high glucose DMEM medium supplemented with 10, 20 and 40 ng / mL of IFNy (R&D Systems, Minneapolis, USA) for 72h. Subsequently, the conditioned medium of MSC was collected to determine the enzymatic activity of IDO. For this purpose, the N-formyl-kinurenine concentration in the medium was quantified using a colorimetric assay (Saulnier N, Loriau J, Febre M, Robert C, Rakic R, Bonte T, Buff S, Maddens S. 2016. Canine placenta: A promising potential source of highly proliferative and immunomodulatory mesenchymal stromal cells? Vet Immunol Immunopathol 171, 47-55) and comparing it with a D-Quinurenine concentration curve (Sigma-Aldrich, USA). Once collected, the conditioned medium was centrifuged at 750 g for 5 min and filtered through pores of 0.22 $\mu$m. A 200 $\mu$l aliquot of MSC conditioned medium was mixed with 100 ml of 30% trichloroacetic acid (Sigma-Aldrich, USA) and the mixture was incubated at 50 °C for 30 minutes. Once centrifuged at 15,000 g for 1 min, 100 $\mu$l of supernatant was transferred to each well of a 96-well flat-bottom plate and 100 $\mu$l of Ehrlich reagent (100 mg of p-dimethylamino benzaldehyde diluted in 5 ml of acid glacial acetic). Next, the optical density at 450 nm was determined with a microplate reader (Model 3550, Bio-Rad, Arizona, USA). See Figure 12B.

BM-MSCs and AT-MSCs treated with 20 and 40 ng / mL of IFNy expressed (4.1, 1, 6 and 2.5, 1.4 times, respectively) higher ($P< 0.05$) levels of mRNA of IL-6 in relation to the control MSC treated with 10 ng / mL of IFNy (Figure 12). On the other hand, BM-MSCs treated with 40 ng / mL IFNy expressed 3.24 times higher ($P< 0.05$) levels of PGE2 mRNA compared to BM-MSCs treated with lower concentrations of IFNy and MSC not treated with IFNy. TGF$\beta$1 and IL-10 mRNA levels were not different ($P> 0.05$) when compared between MSCs treated with different concentrations of IFNy. When BM-MSCs and AT-MSCs were treated with IFNy, the expression of IDO mRNA was activated. For BM-MSCs and AT-MSCs treated with 40 ng / mL IFNy, an increase ($P <0.05$) of mRNA expression was detected compared to MSCs treated with 10 ng / mL IFNy (2.05 and 3.04 vs. 1 time, respectively). However, no significant differences were detected between both cell lines. In the conditioned media obtained from the same cell cultures, the concentration of kinurenine was determined through a colorimetric reaction with Ehrlich's reagent (Figure 12B). The concentration of quinurenine in conditioned medium of MSCs treated with 10 ng / mL of IFNy (5.1 and 6.2 ng / mL for BM-MSCs and AT-MSCs, respectively) was higher ($P <0.05$) in comparison to MSCs not treated with IFNy (1.5 and 1.4 ng / mL for MSC-MO and MSC-TA, respectively). In addition, higher ($P <0.05$) concentrations of kynurenine were detected in conditioned medium of MSCs treated with 40 ng / mL of IFNy (8.2 and 8.9 ng / mL for MSC-MO and MSC-TA, respectively) compared to 10 ng / mL IFNy and MSC not treated with IFNy. Likewise, no differences in kinurenine concentration were detected between both cell lines.

Additionally, the expression levels of major histocompatibility complex immunogenicity genes I and II (MHC-I and II) and T lymphocyte costimulatory molecules (CD80 and CD86) were quantified (Figure 13A). The mRNA levels of MHC-I, CD80 and CD86 in the three cell lines were not different ($P> 0.05$) between MSC-MO and MSC-TA. However, MHC-I I mRNA levels in BM-MSCs were 3.51 times higher ($P <0.05$) compared to the fibroblast control. A higher ($P <0.001$) proportion (79.4%) of the PBMCs expressed MHC-II in relation to BM-MSCs (26.8%), AT-MSCs (8.6%) and FBs (9.9%) (Figure 13B). Likewise, a higher ($P <0.001$) proportion of the BM-MSCs population was positive for MHC-I I compared to MSC-TA.

These results indicate that both MSC cell types express immune mediating factors that could exert an immunomodulatory role for therapeutic treatment in cattle. Considering these results, it is possible to conclude that the immunogenicity potential of MSCs is dependent on the source of tissue origin, since BM-MSCs present a higher expression of MHC-I I compared to AT-MSCs, which could determine a higher Immune rejection in a potential application in allogeneic therapy.

*Example 6 Determination of the lymphocytic activation potential of fetal bovine MSCs by mixed culture*

The effect of conditioned medium of BM-MSCs and AT-MSCs on the proliferation of T lymphocytes activated by alloantigens in vitro was evaluated by means of mixed leukocyte reaction (MLR) (Bocharov G, Luzyanina T, Cupovic J, Ludewig B. 2013. Asymmetry of cell division in CFSE-based lymphocyte proliferation analysis. Frontiers in immunology 264, 1-7). Bovine peripheral blood mononuclear cells (PBMC) were isolated by density gradient (Flistopaque-1077, Sigma-Aldrich). A 15-mL sample of bovine blood was drawn through the coccygeal vein using heparin vacutainer tubes. Then the blood was mixed with PBS in equal parts (1: 1). On the other hand, in a 50 mL falcon tube, 15 mL of Histopaque (Sigma-Aldrich) were added and the blood solution with PBS (30 mL) was added without mixing. Subsequently, the tube was centrifuged at 900 g without brake for 35 minutes. In this way, a separation into four phases was achieved, plasma plus PBS, PBMCs, Histopaque and in the background granulocytes and red blood cells. With a pipet, the PBMC phase was extracted, then it was washed with 10 mL of PBS and centrifuged twice at 1000 g for 10 minutes, to then be re-suspended in supplemented RPMI. In order to perform the purification of peripheral blood lymphocytes (PBLs), the PBMCs were re-suspended in RPMI 1640 medium with 10% FBS (Hyclone Laboratories), 100 $\mu$g / mL of amphotericin B, 100 $\mu$g / mL of streptomycin and 100 IU / mL of penicillin in a 6-well plate (Costar 3516, USA) and incubated at 38 °C, with 5% $CO_2$ for 2 h. After that, the cells not adherent to the plastic were removed and washed with PBS. The PBL was centrifuged and the pellet was resuspended in PBS (Quah et al., 2007). Previously, cell counting was performed in a Neubauer chamber using trypan blue to determine cell viability. Subsequently, the cell concentration was adjusted to $2 \times 10^6$ cells per mL. The PBLs was subsequently labelled with a fluorescent carboxyfluorescein succinimidyl ester (CFSE) probe using a cell proliferation kit (CellTrace, Thermo Fisher, California, USA). Each vial of stock solution (5 mM) was diluted with 18 pL of DMSO and from this dilution 2 $\mu$L (10 pM) were used to mark a total of 1 $\times 10^6$ PBLs in 1 mL of PBS in a suspension that was incubated for 20 min at 37 °C by gentle shaking, protected from light (Quah et al., 2007; Bocharov G, Luzyanina T, Cupovic J, Ludewig B. 2013. Asymmetry of cell division in CFSE-based lymphocyte proliferation analysis. Frontiers in immunology 264, 1-7). Subsequently to this solution a five-fold proportion of medium with SFB was added and it was incubated again for 5 minutes, this process is carried out to eliminate the free dye that is in the solution. It was then centrifuged and the pellet was resuspended in culture medium for subsequent tests. The PBMCs was treated with mitomycin C to inhibit DNA synthesis, suppressing its proliferation but keeping the cells viable and capable of stimulating the proliferation of PBLs. For this, 1 x $10^6$ PBMCs were previously treated with 50 $\mu$g / mL of mitomycin C at 37 °C for 2 h and then they were washed three times with PBS with 5% FBS. To induce alloproliferation, PBLs labeled with CFSE (CellTrace) as responder were co-incubated with PBMCs as allogeneic stimulator treated with mitomycin C in a 96-well U-bottom culture plate for 5 days (Bocharov G, Luzyanina T, Cupovic J , Ludewig B. 2013. Asymmetry of cell division in CFSE-based lymphocyte proliferation analysis. Frontiers in immunology 264, 1-7). Different stimulator and responder ratios (1: 1, 1: 2 and 1: 4) were incubated with the aim of standardizing the method for cattle and working with the proportion that stimulates the proliferation of PBLs to the highest level. As a proliferation control, PBMCs plus PBLs without conditioned medium was used. The cells were subsequently recovered for proliferation analysis by flow cytometry. The data were analyzed using a Gallios cytometer kit (Beckman Coulter, Brea, California, USA) and the Cell Quest program. See Figure 14. The proliferation of bovine PBL is lower in the presence of conditioned medium of both cell types, both pre-activated or not with IFN$\gamma$. These results indicate that fetal bovine MSCs possess immunomodulatory capacity and that their immunomodulatory mechanism could be mediated through the secretion to the culture medium of paracrine factors IDO, IL-6, TGF$\beta$1, IL-10 and PGE2, which inhibit cell proliferation. CD4 + (Th1 and Th17) and CD8 + T cells, while they induce CD4 + (Th2) and Treg T cell generation.

*Example 7 Migration potential and stimulation of angiogenesis in vitro of MSCs derived from bone marrow and bovine fetal adipose tissue*

The "scratch" migration test was used to determine the migratory capacity of MSCs, using fetal FBs as biological controls (Figure 15). For this test, 44 x $10^3$ cells / $cm^2$ were cultured until reaching 80% confluence. After 24 hours, the "scratch" was performed on the monolayer of each cell line and the migration area was quantified at time 0 and after 24 hours using ImageJ software. Using scratch test, a higher (P <0.05) percentage of in vitro migration was determined in BM-MSCs cultures compared to FBs. However; the migration percentages were not different (p> 0.05) between BM-MSCs and AT-MSCs.

Transwell migration analysis was used to determine the potential for cell migration in response to chemotactic stromal cell-derived factor-1 (SDF-1) (Figure 16). For this analysis, 25 x $10^3$ cells / mL were cultured for 24 hours, in response to SDF-1 using 5% FBS and DMEM medium as controls. The transwell assay allowed to determine that a higher (p <0.05) number of migrating cells in cultures treated with DMEM supplemented with 5% FBS. However; no significant differences were detected between cell lines. The analysis of the expression of genes associated with cell migration was performed by Q-PCR in BM-MSCs, AT-MSCs and FBs (Figure 17).

The potential between BM-MSCs and AT-MSCs was similar, which can be associated to similar expression levels of the CXCR4 receptor. The angiogenic potential of the MSC and FB conditioned medium was evaluated using the in vitro endothelial cell tubule formation assay (Figure 18). To carry out this test, $5 \times 10^5$ cells / mL of BM-MSCs, AT-MSCs and FBs were cultured in 75 $cm^2$ culture bottles for 7 days, changing the medium every 72 hours. Once they reached 80% confluence, the culture medium was removed and DMEM without SFB was added in order to produce conditioned medium. Once collected, the conditioned medium was concentrated 10 times by centrifugation for 30 minutes at 1800 rpm in Amicon Ultra Centrifugal filters (Merck Millipore, Tullagreen) and was subsequently frozen at -20 °C until use. For the tubule formation assay, 100 ml of matrigel without growth factors (Corning, Bedford) was pipetted into 24-well plates for the establishment of a basement membrane matrix. The matrigel was gelled at 38 °C for 30 minutes. Subsequently, the endothelial cells (1.8 x $10^5$ cells / well) previously extracted were seeded and cultured on top of the gelled Matrigel membrane. These cells were then resuspended in the concentrated medium of BM-MSCs, AT-MSCs and FBs and seeded on the Matrigel layer. After 6 hours of incubation at 38 °C, the tubules formed were quantified by obtaining digital images obtained with 4x magnification and evaluation using Image J software (NIMH). The concentrated AT-MSC conditioned medium induced greater (P <0.05) tubule formation, compared to BM-MSCs, FBs and their DMEM controls with 5% SFB and DMEM (Figure 18A and B). Furthermore, a higher (P <0.05) expression of VEGF (Vascular endothelial growth factor) was quantified in AT-MSCs compared to the other cell lines (Figure 18C). In comparison, ANGPT1 expression was higher (p <0.05) in BM-MSCs and FBs compared to AT-MSCs.

*Example 8: Evaluation of safety parameters including clinical variables, lymphocyte activation and cytokine production in trials of intramammary administration of an allogeneic therapy of fetal bovine MSCs in healthy Holstein Friesian heifers.*

A safety test was carried out in healthy cows in order to determine the effect of inoculation, via the teat canal, of an MSC suspension of allogeneic origin on the clinical parameters including hemogram and biochemical profile in Holstein Friesian cows. MSCs were isolated from adipose tissue collected from abattoir-derived fetuses previously tested for Brucella abortus (Rose Bengal), Leptospirosis (Microagglutination), Bovine Viral Diarrhea (Elisa) and Infectious Bovine Rhinotracheitis (Elisa). Only fetuses negative for these diseases were used as donors (n = 14). The safety test was carried out in a dairy farm of 997 lactating cows that has been declared free of Brucellosis, Tuberculosis and Bovine Leukosis. Clinically healthy Holstein Friesian heifers (n = 8) of age (15.4 $\pm$ 1.9 months) with weight (445.6 $\pm$ 37.3 kg) were used. A total of 25 x $10^6$ MSCs were suspended in 3,000 Ringer Lactate and this suspension was inoculated into each of two mammary quarters of each cow. A control suspension of 3 mL of Lactated Ringer was inoculated in each of the two remaining mammary quarters of each cow. The order of administration per room was determined randomly. Two doses of MSCs were administered 10 days apart (dose 1 = day 0 and dose 2 = day 10) and clinical parameters (heart rate, respiratory rate, ruminal rate, rectal temperature and mammary gland temperature) were evaluated daily from the day 0 to day 20 (Table 4).

In addition, blood samples were obtained from the coccygeal vein every 5 days from day 0 to day 20, for hemogram analysis and biochemical profile. Additionally, lymphocyte and monocyte populations were isolated from peripheral blood for analysis of CD3, CD4, CD8, and FoxP3 markers by quantitative PCR.

**Table 4.**

| Clinical, hematological, and biochemical profile parameters in Holstein Friesian heifers inoculated with two doses of adipose-derived fetal bovine MSC intramammary. | | |
|---|---|---|
| Variable | Expected value | Obtained value |
| HR (bpm) | 60 adult 120 calf | 77.7 $\pm$ 4.5 |
| RR (bpm) | 24 adult 48 calf | 45.5 $\pm$ 9.4 |
| Ruminal frequency (in 2 min) | 3 | 3.2 $\pm$ 0.5 |
| Rectal Temp. | 38-39 adult y 39-40.5 ternero | 38.9 $\pm$ 0.3 |
| Termography FL(°C) | 33.45-34.1 | 31.8 $\pm$ 2.2 |
| Termography BL(°C) | 33.45-34.1 | 32.2 $\pm$ 2.1 |
| Termography FR (°C) | 33.45-34.1 | 32.1 $\pm$ 1.8 |
| Termography BR (°C) | 33.45-34.1 | 32.3 $\pm$ 1.7 |
| Erythrocytes (mm3) | 5,000,000- 10,000,000 /mm$^3$ | 6,213,214 $\pm$ 138,603 |
| VGA (%) | 24-46% | 28.9 $\pm$ 0.7 |
| Hemoglobin (g/dL) | 8-15 g/dL | 10 $\pm$ 0.3 |
| Leukocytes (mm$^3$) | 4,000-12,000/ mm$^3$ | 23,491 $\pm$ 9,647 |
| Eosinophils (mm$^3$) | <2,400 mm$^3$ | 267.2 $\pm$ 113.7 |
| Basophils (mm$^3$) | <200 mm$^3$ | 0 |
| Myelocytes (mm$^3$) | <0 mm$^3$ | 0 |
| Juveniles (mm$^3$) | <0 mm$^3$ | 0 |
| Baciliformes (mm$^3$) | <120 mm$^3$ | 0 |
| Segmented (mm$^3$) | 600-4,000 mm$^3$ | 7,919 $\pm$ 538 |
| Lymphocytes (mm$^3$) | 2,500-7,500 mm$^3$ | 11,328 $\pm$ 2,758 |
| Monocytes (mm$^3$) | 25-850 mm$^3$ | 0 |
| N° Platelets(mm$^3$) | 100,000-800,000 mm$^3$ | 259,507 $\pm$ 45,106 |
| TPP (g/dL) | 6.5-7.5 g/dL | 6.7 $\pm$ 0.03 |

(continued)

| Clinical, hematological, and biochemical profile parameters in Holstein Friesian heifers inoculated with two doses of adipose-derived fetal bovine MSC intramammary. | | |
|---|---|---|
| Variable | Expected value | Obtained value |
| Albumins (g/dL) | 3.0-3.6 g/dL | $3.21 \pm 0.04$ |
| Globulins (g/dL) | 3.0-3.5 g/dL | $3.48 \pm 0.06$ |
| A/G Index | 0.86-1.2 | $0.93 \pm 0.03$ |
| Cholesterol (mg/dL) | 80-120 mg/dL | $106.29 \pm 8.5$ |
| Calcio (mg/dL) | 9.7-12.4 mg/dL | $8.64 \pm 0.1$ |
| Phosphorus (mg/dL) | 5.6-6.5 mg/dL | $6.57 \pm 0.3$ |
| Glucose (mg/dL) | 45-75 mg/dL | $68.08 \pm 18.1$ |
| BUN (mg/dL) | 20-30 mg/dL | $14.1 \pm 5.9$ |
| Creatinine (mg/dL | 1-2 mg/dL | $0.81 \pm 0.04$ |
| CK total (U/L) | 14-107 U/L | $265.67 \pm 38$ |
| AST (U/L) | 78-132 U/L | $71.78 \pm 4.2$ |
| GGT (U/L) | 6.1-17.4 | $21.5 \pm 0.8$ |
| AP (U/L) | <488 U/L | $352.72 \pm 25.9$ |
| Total Bilirubin (mg/dL) | 0.01-0.5 mg/dL | $0.16 \pm 0.04$ |
| Fibrinogen (mg/dl) | 300-700 mg/dL | $312.74 \pm 16.8$ |

The analysis of the data indicates that the administration of a $25 \times 10^6$ suspension of allogeneic MSCs via the teat canal, in two doses separated by ten days, does not significantly affect the clinical parameters, hemogram variables and biochemical profile and expression of markers. of CD4 + and CD8 + lymphocyte populations or on the mRNAs of the CD25 and CD62L lymphocyte activation molecules (Figure 19). These results indicate that MSCs do not induce significant immunogenic response in cows and therefore it is safe for an effectiveness test in models with induced mastitis.

## Claims

1. Composition for the treatment of mastitis in milk-producing animals **characterised in that** it comprises fetal mesenchymal stem cells (MSC) of allogenic origin, wherein said MSC are obtained from bone marrow.

2. The composition according to claim 1 **characterized in that** said composition is administered via intramammary route.

3. Method of treatment of mastitis in milk-producing animals comprising administering a composition comprising fetal mesenchymal stem cells (MSC) of allogenic origin, wherein said MSC are obtained from bone marrow.

4. The method according to claim 3 **characterized in that** said milk-producing animal is a bovine.

5. The method according to claim 3 **characterized in that** said composition is administered via intramammary route.

6. The method according to claim 3 **characterized in that** said composition is administered in combination with an antibiotic.

7. The method according to claim 6 **characterized in that** said antibiotic is selected from cephalosporins, penicillins, streptomycins, tetracyclines or a combination thereof.

8. Use of a composition comprising fetal mesenchymal stem cells (MSC) of allogenic origin, wherein said MSC are obtained from bone marrow **characterized in that** said composition is useful in the treatment of mastitis in a milk-producing animal.

9. Use of a composition comprising fetal mesenchymal stem cells (MSC) of allogeneic origin and obtained from bone marrow, in combination with an antibiotic **characterized in that** said composition is useful in the treatment of mastitis in a milk-producing animal.

10. The use according to claim 9 **characterized in that** said antibiotic is selected from cephalosporins, penicillins, streptomycins, tetracyclines or a combination thereof.

11. Use of a composition comprising fetal mesenchymal stem cells (MSC) of allogenic origin wherein said MSC are obtained from bone marrow, **characterised in that** said composition is useful in the preparation of a medicament for the treatment of mastitis in a milk-producing animal and in the mammary regeneration of said animal suffering from mastitis.

12. Use of a composition comprising fetal mesenchymal stem cells (MSC) of Allogeneic origin, where said MSC are obtained from bone marrow, in combination with an antibiotic **characterized in that** said composition is useful in the preparation of a medicament for the treatment of mastitis in a milk-producing animal and in the mammary regeneration of said animal suffering from mastitis.

13. The use according to claim 12 **characterized in that** said antibiotic is selected from cephalosporin, penicillin, streptomycins, tetracyclines or a combination thereof.

Application of an intramammary suspension of MSC derived from bovine fetuses for the treatment of mastitis in cows

Figure 1

Figure 2

Figure 3

Figure 4

Figure 5

Figure 6

Figure 7

Figure 8

Figure 9

Figure 10

Figures 11A and 11B

Figures 12A-12F

A

B

| Marker/Cell type | PBMC | BM-MSC | AT-MSC | FB |
|---|---|---|---|---|
| MHC-II (%) | 79.4 ± 0.9[a] | 26.8 ± 1.3[b] | 8.6 ± 0.6[c] | 9.9 ± 6.5[c] |

Figures 13A and 13B

| Treatment | PC | BM-MSC | AT-MSC | BM-MSC IFNγ | AT-MSC IFNγ |
|---|---|---|---|---|---|
| PBL (%) | 34 ± 1.9[a] | 20.2 ± 2.4[b] | 15.4 ± 1.9[b] | 21.1 ± 2[b] | 19.7 ± 1.8[b] |

Figure 14

**BM-MSC**

**AT-MSC**

**FB**

0 h

24 h

Figure 15A

Figure 15B

FBS            SDF-1            DMEM

Figure 16A

Figure 16B

Figure 17

Figure 18A

Figura 18B

Figure 18C

Figures 19A-19D

## INTERNATIONAL SEARCH REPORT

| International application No. |
|---|
| PCT/CL2018/050046 |

| A. CLASSIFICATION OF SUBJECT MATTER |
|---|
| CIP: A61K35/28, A61P31/04 (2018.01) |
| According to International Patent Classification (IPC) or to both national classification and IPC |

| B. FIELDS SEARCHED |
|---|
| Minimum documentation searched (classification system followed by classification symbols) |
| CIP: A61K35/28, A61P31/04 |
| Documentation searched other than minimum documentation to the extent that such documents are included in the fields searched |
| |
| Electronic data base consulted during the international search (name of data base and, where practicable, search terms used) |
| STN (EMBASE, AGRICOLA, USPATFULL), PATENTSCOPE, ESP@CENET, GOOGLE SCHOLAR |

| C. DOCUMENTS CONSIDERED TO BE RELEVANT | | |
|---|---|---|
| Category* | Citation of document, with indication, where appropriate, of the relevant passages | Relevant to claim No. |
| X | CAHUASCANCO, Berly. Determinación del potencial antibacteriano contra Staphylococcus aureus de células madre mesenquimáticas derivadas de médula ósea y tejido adiposo fetal bovino. Santiago, Chile: Universidad de Chile - Facultad de Ciencias Veterinarias y Pecuarias, 2017. [Recuperado el 10-10-2018]. Retrieved from: < http://repositorio.uchile.cl/handle/2250/144991> "Non-detrimental document" The whole document. | 1-13 |
| X | ALVARADO, R. Células troncales como potencial tratamiento para la Mastitis Bovina. En: Noticias d e la Facultad de Ciencias Veterinarias y Pecuarias de la Universidad de Chile. Comunicacione s Campus Sur. Publicado online: 28 de julio de 2017. [Recuperado el 10-10-2018]. Recuperado de: <http://uchile.cl/v135466> "Non-detrimental document" The whole document. | 1-13 |
| X<br>----------<br>Y | PERALTA, O. A. et al. In vitro therapeutic potential of fetal bovine mesenchymal stem cells: develop ment of a novel treatment for bovine mastitis. En: Poster Abstract Book, The Global Stem Cell Ev ent, ISSCR Annual Meeting, Boston, USA, 14-17 June, 2017. [Recuperado el 08-10-2018]. Recupe rado de: <http://www.isscr.org/meetings-events/annual-meetings/past-meetings/isscr-2017-b oston/program-abstracts> "Non-detrimental document" The whole document. | 1-10<br>----------------<br>11-13 |

| [X] Further documents are listed in the continuation of Box C. | [X] See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 10/10/2018 | 15/11/2018 |

| Name and mailing address of the ISA/ | Authorized officer |
|---|---|
| INAPI, Av. Libertador Bernardo O'Higgins 194, Piso 17, Santiago, Chile | GARRIDO GAMBOA, Carolina |
| Facsimile No. | Telephone No. |

Form PCT/ISA/210 (second sheet) (January 2015)

## INTERNATIONAL SEARCH REPORT

International application No.

PCT/CL2018/050046

C (Continuation). DOCUMENTS CONSIDERED TO BE RELEVANT

| Category* | Citation of document, with indication, where appropriate, of the relevant passages | Relevant to claim No. |
|---|---|---|
| X ------- Y | HUAMAN, Olger. Evaluación del potencial de proliferación, inmunomodulación e inmunogenicidad in vitro de células madre mesenquimáticas derivadas de médula ósea y tejido adiposo fetal bovino. Santiago, Chile: Universidad de Chile - Facultad de Ciencias Veterinarias y Pecuarias, 2017. [Recuperado el 10-10-2018). Retrived from: <http://repositorio.uchile.cl/handle/2250/145004> "Non-detrimental document" | 1, 2 ----------- 11-13 |
| X | Pages 26 y 27. DUEÑAS, F. et al. Hepatogenic and neurogenic differentiation of bone marrow mesenchymal stem cells from abattoir-derived bovine fetuses. BMC Veterinary Research, 2014, 10:154, 13 pp. DOI:10.1186/1746-6148-10-154 "Quoted in the application" Page 2, right column , first paragraph; page 9, left column, last paragraph until right column , first paragraph. | 1, 2 |
| X | JERVIS, Miguel. Determinación del potencial de migración y estimulación de angiogénesis in vitro de células células madre mesenquimáticas derivadas médula ósea y tejido adiposo fetal bovino. Santiago, Chile: Universidad de Chile - Facultad de Ciencias Veterinarias y Pecuarias, 2017. [Recuperado el 10-10-2018]. Retrived from: < http://repositorio.uchile.cl/handle/2250/145010> "Non-detrimental document" | 1, 2 |
| X | Pages 25 y 26. CORTES, Y. et al. Isolation and multilineage differentiation of bone marrow mesenchymal stem cells from abattoir-derived bovine fetuses. BMC Veterinary Research. 2013;9:133, 14 pp. DOI:10.1186/1746-6148-9-133. "Quoted in the application" page 2; pages 3, left column, first paragraph; page 10, left column ,thirdy paragraph. | 1, 2 |
| X | OKAMURA, L. H. et al. Myogenic Differentiation Potential of Mesenchymal Stem Cells Derived from Fetal Bovine Bone Marrow, Animal Biotechnology, 2018, 29 (1 ):1-11. Published online: 07 Mar 2017. DOI : 10.1080/10495398.2016.1276926 Page 2, left column, last paragraph untill right column, first paragraph | 1, 2 |
| A | US 2014/0134140 A1 (CASE WESTERN RESERVE UNIVERSITY) 15 de mayo de 2014. "Quoted in the application" Paragraphs [0004]-[0010], [0069]-[0071], [0081], [0086], [0087]; claims 1 , 3, 9, 10, 12, 13, 14. | |
| A | ALCAYAGA-MIRANDA, F. Combination therapy of menstrual derived mesenchymal stem cells and antibiotics ameliorates survival in sepsis. Stem Cell Research & Therapy, 2015, 6:199, 13 pp. DOI:10.1186/s13287-015-0192-0. The whole document. | |
| A | JOHNSON, V. et al. Activated Mesenchymal Stem Cells Interact with Antibiotics and Host Innate Immune Responses to Control Chronic Bacterial Infections. Scientific Reports. 2017;7:9575, 18 pp. DOI:10.1038/s41598-017-08311-4. The whole document. | |
| A | SHARMA, N. & JEONG, D. K. Stem cell research: A Novel Boulevard towards Improved Bovine Mastitis Management. International Journal of Biological Sciences. 2013; 9(8):818-829. DOI:10.7150/ijbs.6901. "Quoted in the application" The whole document. | |
| A | SUTTON, M. T. et al. Antimicrobial Properties of Mesenchymal Stem Cells: Therapeutic Potential for Cystic Fibrosis Infection, and Treatment. Stem Cells International, 2016, vol. 2016, Article ID 5303048, 12 pp. DOI: 10.1155/2016/5303048. The whole document. | |

Form PCT/ISA/210 (continuation of second sheet) (January 2015)

INTERNATIONAL SEARCH REPORT
Information on patent family members

International application No.

PCT/CL2018/050046

US 2014/0134140 A1          15-05-2014     NONE

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20140134140 A1

**Non-patent literature cited in the description**

- **JOSEPH NM ; MORRISON SJ.** Toward an understanding of the physiological function of mammalian stem cells. *Dev Cell,* 2005, vol. 9, 173-183
- **CLARKE MF ; FULLER M.** Stem cells and cancer: two faces of eve. *Cell,* 2006, vol. 124, 1111-1115
- **JAENISCH R ; YOUNG R.** Stem cells, the molecular circuitry of pluripotency and nuclear reprogramming. *Cell,* 2008, vol. 132 (4), 567-582
- **CAPLAN AL.** Mesenchymal stem cells. *J Orthop Res,* 1991, vol. 9 (5), 641-650
- **PITTENGER MF ; MACKAY AM ; BECK SC ; JAISWAL RK ; DOUGLAS R ; MOSCA JD ; MOORMAN MA ; SIMONETTI DW ; CRAIG S ; MARSHAK DR.** Multilineage potential of adult human mesenchymal stem cells. *Science,* 1999, vol. 284 (541 1), 143-147
- **CORTES Y ; OJEDA M ; ARAYA D ; DUEÑAS F ; FERNANDEZ MS ; PERALTA OA.** Isolation and multilineage differentiation of bone marrow mesenchymal stem cells from abattoir-derived bovine fetuses. *BMC Vet Res,* 2013, vol. 9 (133), 1-14
- **GODWIN EE ; YOUNG NJ ; DUDHIA J ; BEAMISH IC ; SMITH RK.** Implantation of bone marrow-derived mesenchymal stem cells demonstrates improved outcome in horses with overstrain injury of the superficial digital flexor tendon. *Equine Vet J,* 2012, vol. 44, 25-32
- **DOMINICI M ; LE BLANC K ; MUELLER I ; SLAPER-CORTENBACH I ; MARINI FC ; KRAUSE DS ; DEANS RJ ; KEATING A ; PROCKOP DJ ; HORWITZ EM.** Minimal criteria for defining multipotent mesenchymal stromal cells. The international society for cellular therapy position statement. *Cytotherapy,* 2006, vol. 8, 315-317
- **YANEZ R ; LAMANA ML ; GARCÍA-CASTRO J ; COLMENERO I ; RAMÍREZ M ; BUEREN J A.** *Stem Cells,* 2006, vol. 24, 2582-2591
- *Stem Cells,* vol. 24, 2582-2591
- **MINTEER DM ; MARRA KG ; RUBIN JP.** Adipose stem cell: biology, safety, regulation and regenerative potential. *Clin Plast Surg,* 2015, vol. 42 (2), 169-179
- **GRONTHOS S ; FRANKLIN DM ; LEDDY FIA ; ROBEY PG ; STORMS RW ; GIMBLE JM.** Surface protein characterization of human adipose tissue-derived stromal cells. *J Cell Physiol,* 2001, vol. 189, 54-63
- **KATZ AJ ; THOLPADY A ; THOLPADY SS ; SHANG H ; OGLE RC.** Cell surface and transcriptional characterization of human adipose-derived adherent stromal (hADAS) cells. *Stem Cells,* 2005, vol. 23, 412-423
- **ZHU Y ; LIU T ; SONG K ; NING R ; MA X ; CUI Z.** ADSCs differentiated into cardiomyocytes in cardiac microenvironment. *Mol Cell Biochem,* 2009, vol. 324 (1), 17-129
- **LIU TM ; MARTINA M ; HUTMACHER DW ; HUÍ JH ; LEE EH ; LIM B.** Identification of common pathways mediating differentiation of bone marrow- and adipose tissue-derived human mesenchymal stem cells into three mesenchymal lineages. *Stem Cells,* 2007, vol. 25, 750-760
- **YOSHIMURA H ; MUNETA T ; NIMURA A ; YOKOYAMA A ; KOGA H ; SEKIYA I.** Comparison of rat mesenchymal stem cells derived from bone marrow, synovium, periosteum, adipose tissue, and muscle. *Cell Tissue Res,* 2007, vol. 327, 449-462
- **BRUNO RD ; SMITH GH.** Role of epithelial stem / progenitor cells in mammary cancer. *Gene Expr,* 2011, vol. 15, 133-140
- **POND AC ; BIN X ; BATTS T ; ROARTY K ; HILSENBECK S ; ROSEN JM.** Fibroblast growth factor receptor signalling is essential for normal mammary gland development and stem cell function. *Stem Cells,* 2013, vol. 32, 178-189
- **CAPUCO AV ; CHOUDHARY RK ; DANIELS KM ; LI RW ; EVOCK-CLOVER CM.** Bovine mammary stem cells: cell biology meets production agriculture. *Animal,* 2012, vol. 6, 382-393
- **CAPUCO AV ; AKERS RM ; SMITH JJ.** Mammary growth in Holstein cows during the dry period: quantification of nucleic acids and histology. *J Dairy Sci,* 1997, vol. 80, 477-487

- **WELLNITZ O ; KERR DE.** Cryopreserved bovine mammary cells to model epithelial response to infection. *Vet Immunol Immunopathol,* 2004, vol. 101, 191-202
- **WEBSTER RA ; BLABER SP ; HERBERT BR ; WILKINS MR ; VESEY G.** The role of mesenchymal stem cells in veterinary therapeutics - a review. *N Z Vet J,* 2012, vol. 60 (5), 265-272
- **SCHNABEL LV ; FORTIER LA ; MCLLWRAITH CW ; NOBERT KM.** Therapeutic use of stem cells in horses: which type, how, and when?. *Vet J,* 2013, vol. 197 (3), 570-577
- **AGGARWAL S ; PITTENGER MF.** Human mesenchymal stem cells modulate allogenic immune cell responses. *Blood,* 2005, vol. 105, 1815-1822
- **CAPLAN AL.** Adult mesenchymal stem cells for tissue engineering versus regenerative medicine. *J Cell Physiol,* 2007, vol. 213, 341-317
- **ZHANG ZY ; TEOH SH ; CHONG MS ; SCHANTZ JT ; FISK NM ; CHOOLANI MA ; CHAN J.** Superior osteogenic capacity for bone tissue engineering of fetal compared with perinatal and adult mesenchymal stem cells. *Stem Cells,* 2009, vol. 27, 126-137
- **LE BLANC K ; TAMMIK L ; SUNDBERG B ; HAYNESWORTH SE ; RINGDÉN O.** Mesenchymal stem cells inhibit and stimulate mixed lymphocyte cultures and mitogenic responses in dependently of the major histocompatibility complex. *Scand J Immunol,* 2003, vol. 57 (1), 1-20
- **LEE RH ; PULIN AA ; SEO MJ ; KOTA DJ ; YLOSTALO J ; LARSON BL ; SEMPRUN-PRIETO L ; DELAFONTAINE P ; PROCKOP DJ.** Intravenous HMSCs improve myocardial infarction in mice because cells embolized in lung are activated to secrete the anti-inflammatory protein TSG-6. *Cell Stem Cell,* 2009, vol. 5, 54-63
- **LEE RH ; SEO MJ ; REGER RL ; SPEES JL ; PULIN AA ; OLSON SD ; PROCKOP DJ.** Multipotent stromal cells from human marrow home to and promote repair of pancreatic islets and renal glomeruli in diabetic NOD / scid mice. *Proc Nati Acad Sci USA,* 2006, vol. 103, 17438-17443
- **NEMETH K ; LEELAHAVANICHKUL A ; YUEN PS ; MAYER B ; PARMELEE A ; DOI K ; ROBEY PG ; LEELAHAVANICHKUL K ; KOLLER BH ; BROWN JM.** Bone marrow stromal cells attenuate sepsis via prostaglandin e (2) -dependent reprogramming of host macrophages to increase their interleukin-10 production. *Nat Med,* 2009, vol. 15, 42-49
- **PAREKKADAN B ; VAN POLI D ; SUGANUMA K ; CARTER EA ; BERTHIAUME F ; TILLES AW ; YARMUSH ML.** Mesenchymal stem cell-derived molecules reverse fulminant hepatic failure. *PLoS One,* 2007, vol. 2, e941
- **TOGEL F ; HU Z ; WEISS K ; ISAAC J ; LANGE C ; WESTENFELDER C.** Administered mesenchymal stem cells protect against ischemic acute renal failure through differentiation-independent mechanisms. *Am J Physiol Renal Physiol,* 2005, vol. 289, F31-F42
- **XU J ; QU J ; CAO L ; SAI Y ; CHEN C ; FIE L ; YU L.** Mesenchymal stem cell-based angiopoietin-1 gene therapy for acute lung injury induced by lipopolysaccharide in mice. *J Pathol,* 2008, vol. 214, 472-481
- **ROJAS M ; XU J ; WOODS CR ; MORA AL ; SPEARS W ; ROMAN J ; BRIGHAM KL.** Bone marrow-derived mesenchymal stem cells in repair of the injured lung. *Am J Respir Cell Mol Biol.,* 2005, vol. 33, 145-152
- **KIDD S ; SPAETH E ; DEMBINSKI JL ; DIETRICH M ; WATSON K ; KLOPP A ; BATTULA VL ; WEIL M ; ANDREEFF M ; MARINI FC.** Direct evidence of mesenchymal stem cell tropism for tumor and wounding microenvironments using in vivo bioluminescent imaging. *Stem Cells,* 2009, vol. 27, 2614-2623
- **PONTE AL ; MARAIS E ; GALLAY N ; LANGONNE A ; DELORME B ; HERAULT O ; CHARBORD P ; DOMENECH J.** The in vitro migration capacity of human bone marrow mesenchymal stem cells: comparison of chemokine and growth factor chemotactic activities. *Stem Cells,* 2007, vol. 25, 1737-1745
- **LEWINSOHN DM ; BARGATZE RF ; BUTCHER EC.** Leukocyte- endothelial cell recognition: evidence of a common molecular mechanism shared by neutrophils, lymphocytes, and other leukocytes. *J Immunol,* 1987, vol. 138, 4313-4321
- **JARVINEN L ; BADRI L ; WETTLAUFER S ; OHTSUKA T ; STANDIFORD TJ ; TOEWS GB ; PINSKY DJ ; PETERS-GOLDEN, M ; LAMA VN.** Lung resident mesenchymal stem cells isolated from human lung allografts inhibit T cell proliferation via a soluble mediator. *J Immunol,* 2008, vol. 181, 4389-4396
- **RASMUSSON I ; LEBLANC K ; SUNDBERG B ; RINGDÉN O.** Mesenchymal stem cells stimulate antibody secretion in human B cells. *Scand J Immunol,* 2007, vol. 65, 336-343
- **LE BLANC K ; TAMMIK L ; SUNDBERG B ; HAYNESWORTH SE ; RINGDÉN O.** Mesenchymal stem cells inhibit and stimulate mixed lymphocyte cultures and mitogenic responses in dependently of the major histocompatibility complex. *Scand J Immunol,* 2003, vol. 57, 11-20
- **GUEST DJ ; SMITH MR ; ALIEN WR.** Monitoring the fate of autologous and allogenic mesenchymal progenitor cells injected into the superficial digital flexor tendon of horses: preliminary study. *Equine Vet,* 2008, vol. 40, 178-181

- **KRAUSE DS ; THEISE ND ; COLLECTOR MI ; HENEGARIU O ; HWANG S ; GARDNER R ; NEUTZEL S ; SHARKIS SJ.** Multi-organ, multi- lineage engraftment by a single bone marrow-derived stem cell. *Cell,* 2001, vol. 105, 369-377
- **FANG X ; NEYRINCK AP ; MATTHAY MA ; LEE JW.** Allogeneic human mesenchymal stem cells restore epithelial protein permeability in cultured human alveolar type ii cells by secretion of angiopoietin-1. *J Biol Chem,* 2010, vol. 285 (2621), 1-26222
- **LEE JW ; FANG X ; KRASNODEMBSKAYA A ; HOWARD JP ; MATTHAY MA.** Concise review: Mesenchymal stem cells for acute lung injury: role of paracrine soluble factors. *Stem Cells,* 2011, vol. 29 (6), 913-919
- **SUTRA L ; POUTREL B.** Virulence factors involved in the pathogenesis of bovine intramammary infections due to Staphylococcus aureus. *J Med Microbiol,* 1994, vol. 40, 79-89
- **TALBOT BG ; LACASSE P.** Progress in the development of mastitis vaccines. *Livestock Prod Sci,* 2005, vol. 98, 101-113
- *International Journal of Biological Sciences,* 2013, vol. 9 (8), 818-829
- **NEELESH SHARMA et al.** *Research Paper Stem Cell Research: A Novel Boulevard towards Improved Bovine Mastitis Management,* 13 June 2013
- Fetal Stem Cells in Farm Animals: Applications in Health and Production PS Yadav. **RK SINGH ; B. SINGH.** Agrie Res. NAAS (National Academy of Agricultural Sciences), 15 October 2011, vol. 1, 67-77
- **CORTES Y ; OJEDA M ; ARAYA D ; DUEÑAS F ; FERNANDEZ MS ; PERALTA OA.** Isolation and multilineage differentiation of bone marrow mesenchymal stem cells from abattoir-derived bovine fetuses. *BMC Vet Res,* 2013, vol. 9 (133), 1-14
- **DUEÑAS F ; BECERRA V ; CORTES Y ; VIDAL S ; SÁENZ L ; PALOMINO J ; DE LOS REYES M ; PERALTA OA.** Hepatogenic and neurogenic differentiation of bone marrow mesenchymal stem cells from abattoir-derived bovine fetuses. *BMC Vet Res,* 2014, vol. 10 (154), 1-13
- **WENZ J ; GARRY F ; BARRINGTON G.** Comparison of disease severity scoring systems for dairy cattle with acute coliform mastitis. *JAVMA,* 2006, vol. 229, 259-262
- **HOGAN SJ ; GONZALEZ NR ; HARMON JR ; NICKERSON CS ; OLIVER PS ; PANKEY JW ; SMITH KL.** Laboratory Handbook on 10 Bovine Mastitis. National Mastitis Council Inc, 1999, 222
- **FRESHNEY RI.** Culture of Animal Cells: A Manual of Basic Technique. 2005, 38-358
- **SCHMITTGEN TD ; LIVAK KJ.** Analyzing real-time PCR data by the comparative C (T) method. *Nat Protoc,* 2008, vol. 3, 1 101-1 108
- **SAULNIER N ; LORIAU J ; FEBRE M ; ROBERT C ; RAKIC R ; BONTE T ; BUFF S ; MADDENS S.** Canine placenta: A promising potential source of highly proliferative and immunomodulatory mesenchymal stromal cells?. *Vet Immunol Immunopathol,* 2016, vol. 171, 47-55
- **BOCHAROV G ; LUZYANINA T ; CUPOVIC J ; LUDEWIG B.** Asymmetry of cell division in CFSE-based lymphocyte proliferation analysis. *Frontiers in immunology,* 2013, vol. 264, 1-7